(19) Europäisches Patentamt
European Patent Office
Office européen des brevets

(11) Publication number: **0 081 312**
**A2**

# (12) EUROPEAN PATENT APPLICATION

(21) Application number: 82306193.2

(22) Date of filing: 22.11.82

(51) Int. Cl.³: C 07 D 487/04
A 61 K 31/395
//(C07D487/04, 209/00, 205/00)

(30) Priority: 09.12.81 GB 8137148

(43) Date of publication of application: 15.06.83 Bulletin 83/24

(84) Designated Contracting States: BE CH DE FR GB IT LI NL SE

(71) Applicant: BEECHAM GROUP PLC
Beecham House Great West Road
Brentford Middlesex(GB)

(72) Inventor: Corbett, David Francis
72E Holmesdale Road
Reigate Surrey(GB)

(72) Inventor: Fell, Stephen Christopher Martin
29 Whiteheart Court North Parade
Horsham Sussex(GB)

(74) Representative: Hesketh, Alan, Dr. et al,
European Patent Attorney Beecham Pharmaceuticals
Great Burgh Yew Tree Bottom Road
Epsom, Surrey, KT18 5XQ(GB)

## (54) Antibiotics, their preparation and use.

(57) Compound of formula (II) and salts and esters are useful for treating bacterial infections:

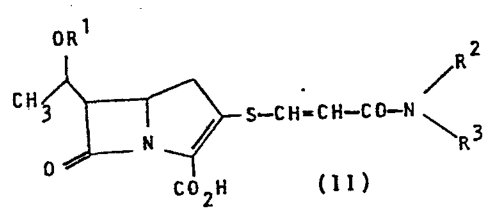

(II)

wherein:

$R^1$ is hydrogen, $-CHO$, or $-SO_3H$ or a pharmaceutically acceptable salt thereof;

$R^2$ and $R^3$ are independently hydrogen, hydrocarbon, hydrocarbylcarbonyl, hydrocarbylcarbonylamino;

or $R^2$ and $R^3$ together with the nitrogen atom to which they are attached form a heterocyclic ring;

$R^2$ and $R^3$ being optionally substituted.

EP 0 081 312 A2

Croydon Printing Company Ltd.

0081312

# ANTIBIOTICS, THEIR PREPARATION AND USE

This invention relates to novel 7-oxo-1-azabicyclo-[3.2.0]heptene derivatives and in particular to such derivatives with C-3 carbamoylvinylthio substituents. This invention further relates to processes for their preparation and to compositions containing them. These derivatives are of use in the treatment of bacterial infections in humans and animals.

European Patent Application Publication Numbers: 0001627, 0001628, 0017992 and 0030032 disclose synthetic antibacterial agents of the formula (I):

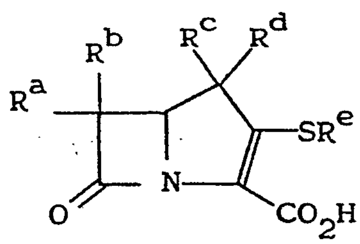

(I)

wherein $R^a$, $R^b$, $R^c$, $R^d$ and $R^e$ may be selected from a wide range of substituents.

BAD ORIGINAL

We have now found that compounds within formula (I) having a C-3 carbamoylvinylthio substituent show superior efficacy, for example antibacterial activity and/or stability than related compounds specifically disclosed in the aforementioned European Patent Applications.

Accordingly the present invention provides the compounds of the formula (II):

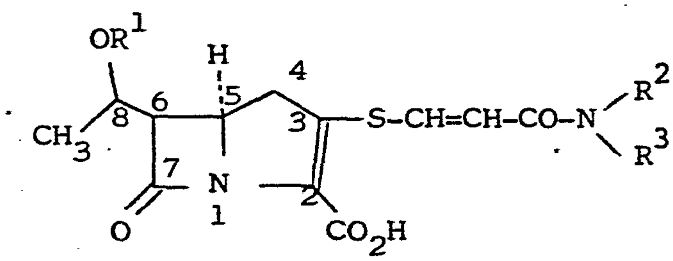

(II)

and pharmaceutically acceptable salts and pharmaceutically acceptable esters thereof, wherein $R^1$ is hydrogen, -CHO or $-SO_3H$ or a pharmaceutically acceptable salt $R^2$ and $R^3$ are independently hydrogen, hydrocarbon, hydrocarbylcarbonyl, or hydrocarbylcarbonylamino; or $R^2$ and $R^3$ together with the nitrogen atom to which they are attached form a heterocyclic ring, $R^2$ and $R^3$ being optionally substituted.

The term 'hydrocarbon' includes groups having up to 18 carbon atoms, suitably up to 10 carbon atoms, conveniently up to 6 carbon atoms. Suitable hydrocarbon groups include $C_{1-6}$ alkyl, $C_{2-6}$ alkenyl, $C_{2-6}$ alkynyl, $C_{3-7}$ cycloalkyl, $C_{3-7}$ cycloalkyl$(C_{1-6})$-alkyl, aryl, and aryl$(C_{1-6})$alkyl.

BAD ORIGINAL

Suitable alkyl groups include straight and branched chain alkyl groups containing from 1 to 6 carbon atoms, such as methyl, ethyl, propyl and butyl. A particular alkyl group is methyl.

Suitable optional substituents for the groups $R^2$ and $R^3$ include $C_{1-6}$ alkyl, amino, hydroxy, $C_{1-6}$ alkoxy, $C_{1-6}$ alkylthio, mercapto, chloro, bromo, fluoro, carboxy and $C_{1-6}$ alkanoyloxy.

Suitably $R^2$ and $R^3$ are selected from hydrogen, $C_{1-6}$ alkyl, $C_{3-10}$ cycloalkyl, aryl ($C_{1-6}$) alkyl, $C_{1-6}$ alkylaminocarbonyl, $C_{3-10}$ cycloalkyl ($C_{1-6}$) alkyl, $C_{3-10}$ cycloalkylaminocarbonyl, halo ($C_{1-6}$) alkyl, amino ($C_{1-6}$) alkyl, or $C_{1-6}$ alkylcarbonyl; or $R^2$ and $R^3$ together represent $C_4$ or $C_5$ alkylene.

More suitably $R^2$ and $R^3$ are independently selected from hydrogen, $C_{1-6}$ alkyl such as methyl, ethyl. or propyl, aryl ($C_{1-6}$) alkyl such as benzyl or phenethyl, $C_{3-7}$ cycloalkyl such as cyclohexyl, or aryl such as phenyl.

In an alternative aspect $R^2$ and $R^3$ together with the nitrogen atom to which they are attached may be joined to form a heterocyclic ring, preferably such a ring contains up to 10 ring atoms and the hetero atoms (other than the nitrogen atom depicted above) being selected from 1 to 3 oxygen, nitrogen or sulphur atoms. In a further aspect such a heterocyclic ring may be fused to a phenyl ring. Thus, suitable examples of $-NR^2R_3$ include optionally substituted succinimido, maleimido, phthalimido, piperidino, pyrrolidinyl piperazino, morpholino and thiamorpholino.

Suitably $R^1$ is $-SO_3H$ or a pharmaceutically acceptable salt thereof such as an alkali metal salt, for example the sodium or potassium salt.

Suitably also $R^1$ is -CHO which compounds are active in their own right but it is also possible that they could be converted to compounds wherein $R^1$ is hydrogen <u>in-vivo.</u>

Preferably $R^1$ is a hydrogen atom.

Thus a preferred group of compounds of this invention is that of the formula (III):

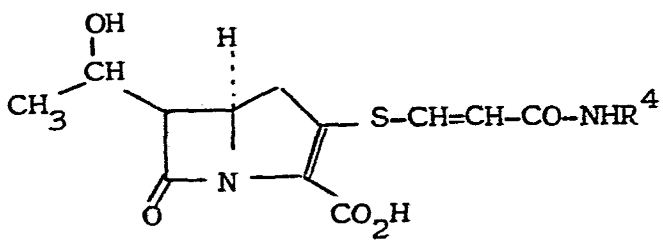

(III)

and pharmaceutically acceptable salts thereof wherein $R^4$ is a hydrogen atom, $C_{1-6}$ alkyl or benzyl.

The compounds of the formulae (II) - (III) may have R or S stereochemistry at the C-8 position (that is the α-carbon atom of the C-6 substitutent) or may be in the form of mixtures thereof.

The compounds of the formulae (II) - (III) may be provided with a cis configuration of the C-5 and C-6 protons, or they may be provided with a trans configuration of the C-5 and C-6 protons. Alternatively they are provided as a mixture of the cis and trans forms.

BAD ORIGINAL

In the compounds of the formulae (II) - (III) the double bond present in the C-3 thio substituent may be present in either the E- configuration or the Z-configuration, or the compounds may be provided as mixtures thereof.

Suitable pharmaceutically acceptable salts include those of the alkali and alkaline earth metals, of these the sodium and potassium salts are preferred. These pharmaceutically acceptable salts may be formed at the C-2 carboxy and/or at the C-6 sulphonato-oxyethyl moiety (if present). Thus compounds of the formula (II) wherein $-R^1$ is $-SO_3H$ or a pharmaceutically acceptable salt thereof, may be in the form of a di- salt such as the di - sodium or di- potassium salt, or may be in the form of a mono-salt of an in-vivo hydrolysable ester, or may be in the form of a mono- salt of an acid or may be in the form of a di- acid.

Non-pharmaceutically acceptable salts of the compounds of the formulae (II) - (III) are also of use as they may be converted to the compounds of the formulae (II) - (III) or a pharmaceutically acceptable salt or ester thereof.

Suitable esters of the compounds of the formulae (II) - (III) include those cleavable by biological methods such as in-vivo hydrolysis, and those cleavable by chemical methods such as hydrogenolysis, hydrolysis, electrolysis or photolysis.

BAD ORIGINAL

Suitably the carboxylic acid is esterified by a group of the sub-formula (a), (b), (c), (d), (e) or (f):

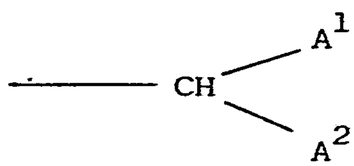

(a)

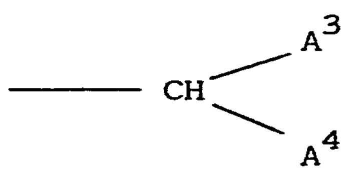

(b)

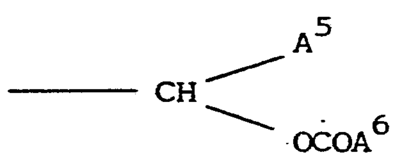

(c)

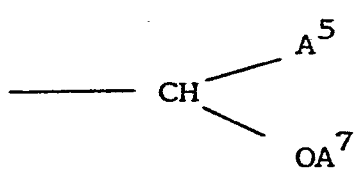

(d)

BAD ORIGINAL

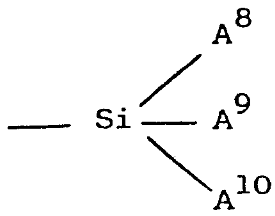

(e)

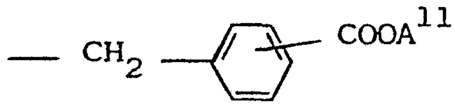

(f)

wherein $A^1$ is a hydrogen atom, $C_{1-6}$ alkanoyl or an alkyl, alkenyl or alkynyl group of up to 3 carbon atoms; $A^2$ is a hydrogen atom or a methyl group; $A^3$ is a phenyl group or a phenyl group substituted by a fluorine, chlorine or bromine atom or a nitro, methyl or methoxy group; $A^4$ is a hydrogen atom or a phenyl group or a phenyl group substituted by a fluorine, chlorine or bromine atom or a nitro, methyl or methoxy group; $A^5$ is a hydrogen atom or a methyl group; $A^6$ is a $C_{1-4}$ alkyl, phenyl or $C_{1-4}$ alkoxy group or $A^5$ is joined to $A^6$ to form a phthalidyl, dimethylphthalidyl or dimethoxyphthalidyl group; $A^7$ is a $C_{1-4}$ alkyl, phenyl, chlorophenyl or nitrophenyl group; $A^8$ is a $C_{1-4}$ alkyl or phenyl group; $A^9$ is a $C_{1-4}$ alkyl or phenyl group; $A^{10}$ is $C_{1-4}$ alkyl; and $A^{11}$ is $C_{1-4}$ alkyl: or $CHA^1A^2$ is a phenacyl or bromophenacyl group.

Favourably $A^1$ is a hydrogen atom or a methyl, ethyl, vinyl or ethenyl group. Favourably $A^2$ is a hydrogen atom. Favourably $A^3$ is a phenyl, p-bromophenyl, p-methoxyphenyl or p-nitrophenyl group. Favourably $A^4$ is a hydrogen atom. Favourably $A^6$ is a methyl, t-butyl

BAD ORIGINAL

or ethoxy group or is joined to $A^5$. Favourably $A^7$ is a methyl group.

Preferred groups of the sub-formula (a) include the methyl, ethyl and acetonyl groups.

Preferred groups of the sub-formula (b) include the benzyl and p-nitrobenzyl groups.

Preferred groups of the sub-formula (c) include the acetoxymethyl, pivaloyloxymethyl, α-ethoxycarbonyloxymethyl and phthalidyl groups.

A preferred group of the sub-formula (d) is the methoxymethyl group.

Preferred groups of the sub-formula (e) include the trimethylsilyl, tert-butyldimethylsilyl and tert-butyldiphenylsilyl groups.

A preferred group of the sub-formula (f) is p-methoxycarbonylbenzyl.

Particularly preferred esterifying groups are the p-nitrobenzyl and phthalidyl groups.

Pharmaceutically acceptable in-vivo hydrolysable esters are those esters which hydrolyse in the human body to produce the parent acid or its salt. Such esters may be identified by administration to a test animal such

BAD ORIGINAL

as a rat or mouse by intravenous administration and thereafter examining the test animal's body fluids for the presence of the compound of the formula (II) or its salt.

Suitable esters of this type include those of sub-formula (c) as hereinbefore defined.

The compounds of this invention may be employed in the treatment of bacterial infection. Thus the present invention also provides a pharmaceutical composition which comprises a compound of the formula (II) - (III) or a pharmaceutically acceptable salt or ester thereof and a pharmaceutically acceptable carrer. Preferably any such ester is <u>in-vivo</u> hydrolysable.

The compositions of this invention may be prepared by conventional methods of preparing antibiotic compositions and in conventional manner may be adapted for oral, topical or parenteral administration.

BAD ORIGINAL

Aptly, the compositions of this invention are in the form of a unit-dose composition adapted for oral administration.

Alternatively the compositions of this invention are in the form of a unit dose composition adapted for administration by injection.

Unit-dose forms according to this invention will normally contain from 50 to 500 mgs of a compound of this invention, for example about 62.5, 100, 125, 150, 200, 250 or 300 mgs. Such compositions may be administered from 1 to 6 times a day or more conveniently 2, 3 or 4 times a day so that the total daily dose for a 70 kg adult is about 200 to 2000 mg, for example about 400, 600, 750, 1000 or 1500 mg.

The compositions of this invention may be used to treat bacterial infection in mammals including humans for example infections of the respiratory tract, urinary tract or soft tissues, or mastitis in cattle. Such compositions may be administered to susceptible gram-positive or gram-negative bacteria such as strains of Staphylococcus aureus, Klebsiella aerogenes and Escherichia coli.

The carriers used in the compositions of this invention may include diluents, binders, disintegrants, lubricants, colours, flavouring agents and preservatives in conventional manner. Thus suitable agents include lactose, starch, sucrose, calcium phosphate, sorbitol, polyvinylpyrrolidone, acacia, gelatin, tragacanth or the like, potato starch or polyvinylpolypyrrolidone, magnesium stearate and sodium lauryl sulphate.

BAD ORIGINAL

Orally administrable forms of the compositions of this invention are most suitably in the form of unit-dose units such as tablets or capsules.

The present invention also provides synergistic pharmaceutical compositions which comprise a pharmaceutical composition as hereinbefore described which also contains a penicillin or a cephalosporin.

Suitable penicillins for inclusion in the compositions of this invention include benzyl penicillin, phenoxymethylpenicillin, ampicillin or a pro-drug therefore, amoxycillin or a pro-drug therefor, carbenicillin of a pro-drug therefor, ticarcillin or a pro-drug therefor, suncillin, sulbenicillin, azlocillin and mezlocillin.

Particularly suitable penicillins for inclusion in orallyadministrable compositions of this invention include ampicillin and its orally administrable pro-drugs, amoxycillin and its orally administrable pro-drugs and orally administrable pro-drugs of carbenicillin. Thus particularly suitable penicillins include ampicillin anhydrate, ampicillin trihydrate, sodium ampicillin, talampicillin hydrochloride, pivampicillin hydrochloride, bacampicillin hydrochloride; amoxycillin trihydrate, sodium amoxycillin; and the sodium salts of the phenyl and 5-indanyl α-esters of carbenicillin.

A preferred penicillin for inclusion in the orally administrable compositions of this invention is amoxycillin trihydrate. A further preferred penicillin for inclusion in the orally administrable compositions of this invention is ampicillin trihydrate.

Particularly suitable penicillins for inclusion

BAD ORIGINAL

in injectably administrable compositions of this invention include injectable salts such as the sodium salt of ampicillin, amoxycillin, carbenicillin and ticarcillin.

A preferred penicillin for inclusion in the injectably administrable compositions of this invention is sodium amoxycillin. A further preferred penicillin for inclusion in the injectably administrable compositions of this invention is sodium ampicillin.

Particularly suitable cephalosporins for inclusion in the compositions of this invention include cephaloridine, cephalexin, cephradine, cefazolin and cephalothin.

A particularly suitable cephalosporin for inclusion in the orally administrable compositions of this invention is cephalexin.

Particularly suitable cephalosporins for inclusions in the injectably administrable compositions of this invention include cefazolin and cephradine, generally as their pharmaceutically acceptable salt such as the sodium salt. Cephaloridine is also particularly suitable for inclusion in injectably administrable compositions.

The weight ratio between compound of this invention and penicillin or cephalosporin is generally from 10:1 to 1:10, for more usually from 5:1 to 1:5 and normally from 3:1 to 1:3.

The penicillin or cephalosporin is generally utilised in its conventionally administered amount.

In another aspect the present invention provides a process for the preparation of a compound of the formula (II) or pharmaceutically acceptable salt or pharmaceutically acceptable ester thereof, which process comprises the reaction of a compound of the formula (IV):

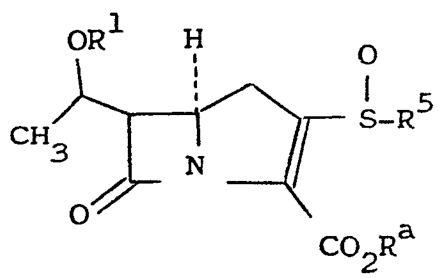

(IV)

wherein $R^1$ is as hereinbefore defined, or protected derivative thereof, $R^a$ is a carboxy-blocking group and $R^5$ is a $C_{1-6}$ alkyl, $C_{3-7}$ cycloalkyl, $C_{3-7}$ cycloalkyl $(C_{1-6})$ alkyl, aryl $(C_{1-6})$ alkyl, heterocyclyl $(C_{1-6})$ alkyl, heteroaryl $(C_{1-6})$ alkyl, heterocyclyl, heteroaryl, aryl, $C_{2-6}$ alkenyl or $C_{2-6}$ alkynyl group, any of such groups being optionally substituted; with a compound of the formula (V):

$$H\text{-}S\text{-}CH\text{=}CH\text{-}CO\text{-}NR^2R^3 \qquad (V)$$

or reactive derivative thereof wherein $R^2$ and $R^3$ are as defined in relation to formula (II); and thereafter if necessary:

i) converting a compound of the formula (II) wherein $R^1$ is -CHO to a compound of the formula (II) wherein $R^1$ is hydrogen,

ii) removing any carboxy-blocking group, or removing any protecting group,

iii) converting the product into a pharmaceutically acceptable salt or pharmaceutically acceptable ester.

Compounds of the formula (IV) are described in our copending U.K. Application Number 8103350.

Suitable protected derivatives of the group $R^1$ are those conventional in the art, for example groups cleavable by hydrolysis such as formate or groups cleavable by hydrogenation such as benzyloxycarbonyloxy and p-nitrobenzyloxycarbonyloxy.

This reaction may be performed in any solvent that is substantially inert during the reaction, for example tetrahydrofuran, dimethylformamide, dioxan, hexamethyl phosphoramide, dimethoxyethane or dimethoxydiethyl ether. Of these solvents dimethylformamide is preferred. Alternatively we have found it useful to use a phase transfer catalysts such as tetra-n-butyl ammonium bromide, cetyl benzyl dimethylammonium chloride and cetyltriethylammonium chloride; suitable solvents include halogenated water-immiscible solvents such as chloroform in the presence of water. The reaction is normally performed at ambient or a depressed temperature, for example 20°C to -70°C, and preferably between 0°C and -50°C. However when using a phase transfer catalyst it is preferably to conduct the reaction between 0°C and ambient temperature. When the thiol of the formula (V) is used the reaction is normally carried out in the presence of a base. Examples of such bases include sodium hydride, sodium hydroxide, sodium alkoxide such as the methoxide, ethoxide or butoxide, sodium amide, potassium hydroxide, potassium alkoxide such as the methoxide ethoxide or butoxide, potassium amide, and trialkylamines such as triethylamine and tri-n-propylamine.

Of these triethylamine is preferred. Preferably the base is present in an amount of at least 0.9 equivalents, more preferably between 1.0 and 1.2 equivalents per mole of the thiol compound. Instead of using a base in the reaction a reactive derivative of the thiol may be used, preferably the reactive derivative is a salt of the thiol, in particular an alkali metal salt such as sodium or potassium. The amount of thiol compound of the formula (V) or reactive derivative thereof is generally between 1.0 and 1.5 moles per mole equivalent of the compound of the formula (IV).

Suitable carboxyl-blocking derivatives for the group $-CO_2R^a$ in formulae (IV) include salts, esters, and anhydride derivatives of the carboxylic acid. The derivative is one which may readily be cleaved at a later stage of the reaction. The salts need not be pharmaceutically acceptable. Suitable salts include inorganic salts, for example metal salts such as silver or mercuric salt, or alkali metal salts such as the lithium or sodium salt, and tertiary amine salts.

Suitable ester-forming carboxyl-blocking groups are those which may be removed under conventional conditions. Such groups for $R^a$ include benzyl, p-methoxybenzyl, 2,4,6-trimethylbenzyl, 3,5-di-t-butyl-4-hydroxybenzyl, benzoylmethyl, p-nitrobenzyl, 4-pyridylmethyl, 2,2,2-trichloroethyl, 2,2,2-tribromoethyl, acetonyl t-butyl, t-amyl, diphenylmethyl, triphenylmethyl, adamantyl, 2-benzyloxyphenyl, 4-methylthiophenyl, tetrahydrofur-2-yl, tetrahydropyran-2-yl, pentachlorophenyl, p-toluenesulphonylethyl, methoxymethyl, a silyl, stannyl or phosphorus-containing group, an oxime radical of formula $-N=CHR^o$ where $R^o$ is aryl or heterocyclic, or an in-vivo hydrolysable ester.

BAD ORIGINAL

The carboxyl group may be regenerated from any of the above esters by usual methods appropriate to the particular $R^a$ group, for example, acid - and base - catalysed hydrolysis, or by enzymically - catalysed hydrolysis, or by hydrogenation. The hydrolysis must of course be carried out under conditions to which the groups on the rest of the molecule are stable.

When it is desired to produce a compound of formula (II) in the form of a free acid or salt by the process of this invention, a compound of formula (IV) is generally employed wherein $R^a$ is a carboxyl-blocking group. For the preparation of a compound of formula (II) in the form of a pharmaceutically acceptable ester, it is convenient to employ a compound of formula (IV) wherein $R^a$ represents the desired ester group.

Preferably the process of this invention is performed on a compound of the formula (IV) wherein $R^a$ is an ester-forming group.

In an alternative aspect the present invention provides a process for the preparation of a compound of the formula (II) or pharmaceutically acceptable salt or pharmaceutically acceptable ester thereof, which process comprises the elimination of the elements of $R^6R^7R^8P{=}O$ from an ester of a compound of the formula (VI):

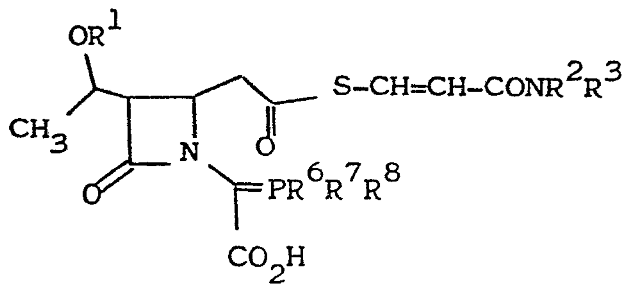

(VI)

wherein $R^1$ is as hereinbefore defined or protected derivative thereof, and $R^2$ and $R^3$ are as hereinbefore defined, and $R^6$, $R^7$ and $R^8$ are independently phenyl or methoxyphenyl, and thereafter if necessary:

i) converting a compound of the formula (II) wherein $R^1$ is -CHO to a compound of the formula (II) wherein $R^1$ is hydrogen.

ii) converting the ester to an acid, pharmaceutically acceptable salt or pharmaceutically acceptable ester.

iii) removing any protecting group.

Suitably this process is performed by heating the ester of the compound of the formula (VI) in an inert solvent, for example at temperatures of 90°C to 120°C and more suitably 100°C to 110°C in a solvent such as toluene, preferably under substantially anhydrous conditions.

The preparation of the compounds of the formula (VI) may be effected by the reaction of a compound of the formula (VII):

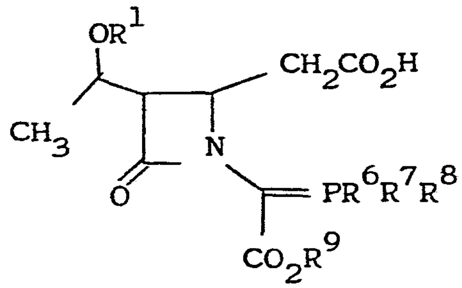

(VII)

wherein $R^1$, $R^6$, $R^7$ and $R^8$ are as defined in relation to formula (VI) and $R^9$ is an esterifying group; with a di-$(C_{1-6})$alkylphosphorochloridate or thionyl chloride and a tri$(C_{1-6})$alkylamine followed by reaction with a derivative of the formula (VIII):

$$L^{\oplus}\ {}^{\ominus}SCH{=}CHCONR^2R^3 \qquad (VIII)$$

wherein $R^2$ and $R^3$ are as hereinbefore defined and $L^{\oplus}$ is a lithium, sodium, silver or thallium (I) cation or an ammonium ion substituted by up to three $C_{1-6}$ alkyl groups.

When $L^{\oplus}$ is a substituted ammonium ion, it is preferably a tertiary ammonium ion, such as the triethylammonium ion. It is conveniently generated <u>in situ</u> by the reaction of a compound of the formula $HSCH{=}CHCONR^2R^3$ with an amine, preferably a tertiary amine.

Preferably $L^{\oplus}$ is a thallium (I) cation or a silver cation.

A particularly suitable di-loweralkylphosphorochloride is diethylphosphorochloridate. A particularly suitable tri-loweralkylamine is triethylamine.

The reaction is generally carried out in an inert organic solvent such as tetrahydrofuran at a non-extreme temperature such as $0^{\circ}C$ to $40^{\circ}C$, for example $15^{\circ}$ to $25^{\circ}C$.

The compounds of the formula (VII) may be prepared by the methods of European Patent Application Publication Numbers: 0008888, 0008514 and 0002564.

In another aspect of this invention there is provided a process for the preparation of a compound of the formula (II) or a pharmaceutically acceptable salt or pharmaceutically acceptable ester thereof, which process comprises the reaction of a compound of the formula (IX):

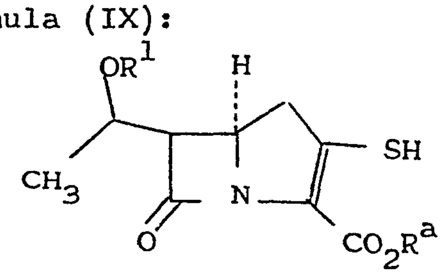

(IX)

or reactive derivative thereof, wherein $R^1$ is as hereinbefore defined or a protected derivative thereof, and $R^a$ is as hereinbefore defined; with a compound of the formula (X):

$$H\text{-}C{\equiv}C\text{-}CO\text{-}NR^2R^3 \qquad (X)$$

wherein $R^2$ and $R^3$ are as hereinbefore defined; in the presence of an acid acceptor, and thereafter if necessary:

i) converting a compound of the formula (II) wherein $R^1$ is -CHO to a compound of the formula (II) wherein $R^1$ is hydrogen,

ii) removing any carboxy-blocking group, or removing any protecting group,

iii) converting the product into a pharmaceutically acceptable salt or pharmaceutically acceptable ester.

Suitable acid acceptors are carbonates and bicarbonates such as anhydrous potassium carbonate. The reaction is generally carried out in a dry polar solvent such as dimethylformamide. Suitably the reaction is performed at a non-extreme temperature, for example -30°C to + 60°C, more suitably -10°C to + 40°C and preferably at ambient temperature.

Compounds of the formula (IX) or a salt or ester thereof may be prepared, for example, by the reaction of an ester of a compound of the formula (XI):

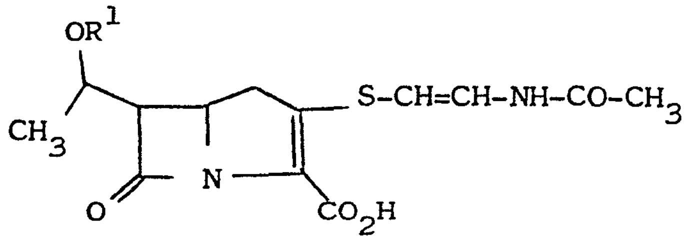

(XI)

wherein $R^1$ is as defined in relation to formula (IX); with a source of hypohalous acid, and thereafter if desired converting the ester to an acid, salt or another ester.

Compounds of the formula (XI) may be obtained from natural sources, or for example where $R^1$ is -CHO may be derived from such naturally occurring compounds by formylation. Alternatively the compounds of the formula (XI) may be prepared synthetically or semi-synthetically.

Suitably the reaction is performed at a non-extreme temperature such as - 15°C to + 25°C, preferably ambient. Solvents suitable in this reaction are inert organic solvents in the presence of moisture, for example moist acetone or dioxan. Suitably the hypohalous acid is hypobromous acid or hypochlorous acid, of these hypobromous acid is preferred. Suitably sources of hypohalous acids include N-bromoacetamide, N-chloroacetamide and N-bromopropionamide.

The following Examples illustrate the invention.

Example 1 a

p-Nitrobenzyl (5R,6R)-3-[2-(Z)-{(N-cyclohexyl,N-cyclohexylaminocarbonyl)aminocarbonyl} ethenylthio]-6-[(S)-1-hydroxyethyl]-7-oxo-1-azabicyclo[3.2.0]hept-2-ene-2-carboxylate

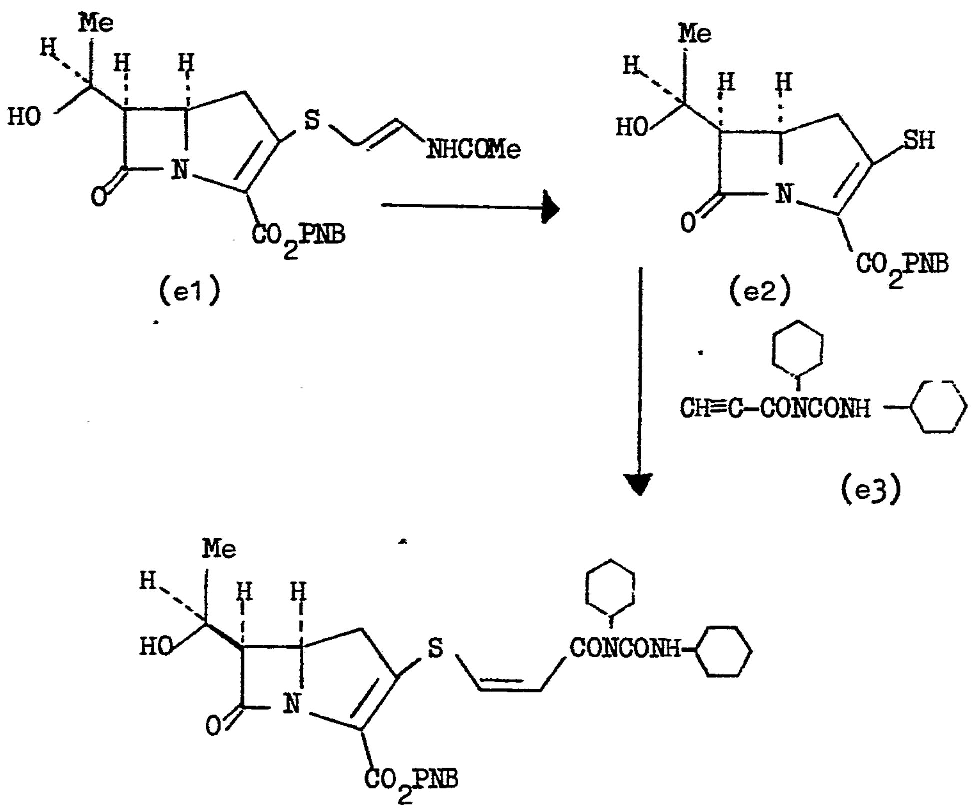

(e1) (e2) (e3) (e4)

A    A solution of the ester (e1) (100 mg) in 15% aqueous dioxan (2 ml) was stirred with a solution of N-bromoacetamide (31 mg) in dioxan (1 ml) at room temperature. After 4.5 min. the solution was diluted with chloroform (20 ml) and washed with 0.05 M pH 7 phosphate buffer (20 ml) and dilute brine (20 ml). The dried ($MgSO_4$) organic solution was evaporated in vacuo to afford a foam which contained the thiol (e2).

B    The foamy product was dissolved in N,N-dimethylformamide (2 ml) and the solution was stirred with the propiolic acid derivative (e3) (120 mg) and anhydrous potassium carbonate (catalytic quantity). After 30 min. the mixture was diluted with ethyl acetate (30 ml) and

the solution was washed with water (3 x 30 ml) and brine (20 ml). The solution was dried ($MgSO_4$) and concentrated in vacuo, and the residue chromatographed on silica gel using 30% hexane-ethyl acetate to elute. Fractions containing the new component were combined and evaporated in vacuo to give a product which, on trituration with ethyl acetate-diethyl ether afforded the title compound (e4) as a white solid (27 mg); $\lambda_{max.}$ (EtOH) 340 and 265 nm; $\nu_{max.}$ (KBr) 1780, 1670-1705br and 1635 $cm^{-1}$; δ (DMF-$d_7$) 1.33 (3H, d, J 6.5 Hz, MeCH), ca. 1.0 - 2.0 (20H, m, cyclohexyl protons), ca. 3.5-3.8 (3H, m, 2 x cyclohexyl CH-N, and 6-CH) 4.10 (2H, m, CHMe and NH or OH), 4.40 (1H, m, 5-CH), 5.18 (1H, br, OH or NH) 5.44 and 5.60 (each 1H, d, J 14 Hz, $CH_2Ar$), 6.34 (1H, d, J 10 Hz,=CH.CO), 7.65 (1H, d, J 10 Hz, SCH=), 7.94 and 8.33 (each 2H, d, J 9Hz, $C_6H_4$-$NO_2$).

The product contained ca. 20% of the corresponding (E)-isomer as shown by signals in the n.m.r. spectrum at δ 6.53 and 7.83 (each d, J 15 Hz, CH=CH).

BAD ORIGINAL

Example 1b

Sodium (5R,6R)-3-[2-(Z)-{(N-cyclohexyl,N-cyclohexylaminocarbonyl)aminocarbonyl}ethenylthio]-6-[(S)-1-hydroxyethyl]-7-oxo-1-azabicyclo[3.2.0]hept-2-ene-2-carboxylate

(e4)

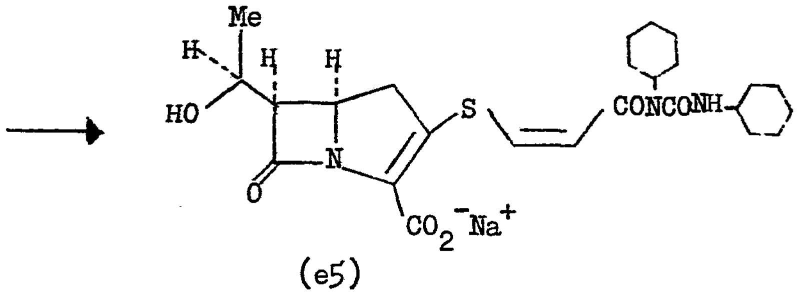

(e5)

A solution of the ester (e4) (10 mg) in 30% aqueous dioxan (10 ml) containing 0.05 M pH 7 phosphate buffer (2 drops) was shaken with 5% Pd-C catalyst (15 mg) under an atmosphere of hydrogen for 2.5 h. The mixture was filtered over Celite, washing the pad well with water. Sodium bicarbonate (3 mg) was added to the solution which was then washed with ethyl acetate (3 x 20 ml) and concentrated *in vacuo* to a volume of *ca.* 20 ml. This solution contained the title sodium salt (e5) with *ca.* 20% of the corresponding (*E*)-isomer; $\lambda_{max.}$ ($H_2O$) 327 nm.

0081312



Example 2a

p-Nitrobenzyl (5R,6R)-3-[(Z)-2-benzylaminocarbonylethenylthio]-6-[(S)-1-hydroxyethyl]-7-oxo-1-azabicyclo[3.2.0]hept-2-ene-2-carboxylate

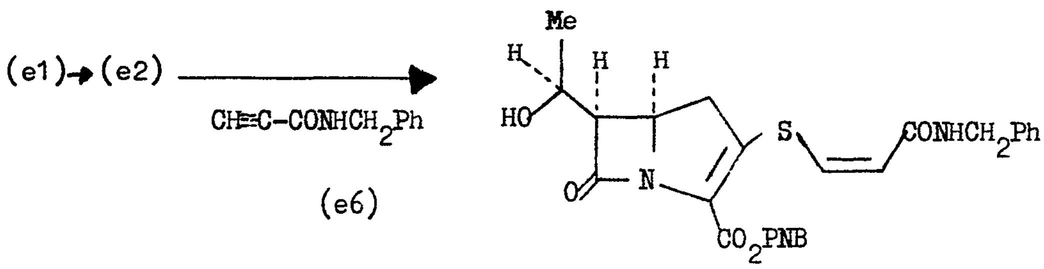


(e7)

A The ester (e1) (200 mg) was converted to the thiol (e2) by the method of Example 1a (A).

B The thiol (e2) was stirred with N-benzylpropiolamide (e6) (150 mg) and potassium carbonate (20 mg) in N,N-dimethylformamide (3 ml) for 40 min. Work up as in Example 1a (B) and chromatography on silica gel using ethyl acetate to elute afforded the title compound (e7) (12 mg) as a pale-yellow solid; $\lambda_{max.}$ (EtOH) 336 (20,590) and 265 nm (13,730); $\nu_{max.}$ (KBr) 1775, 1700 and 1645 $cm^{-1}$; $\delta$ ($CDCl_3$ + acetone $-d_6$) 1.43 (3H, d, J 6.5 Hz, MeCH), 3.25 (1H, dd, J 10 and 18 Hz, 4-CHa), 3.65 (2H, m, 6-CH and 4-CHb), 4.12 (1H, m, CHMe), 4.36 (1H, m, 5-CH), 4.50 (3H, d, J 5.5 Hz, $CH_2NH$), 5.30 and 5.48 (each 1H, d, J 14 Hz, $CH_2Ar$), 6.06 (1H, d, J 10Hz,=CH.CO), 6.71 (1H, br, m, NH), 7.12 (1H, d, J 10 Hz,=CHS), 7.32 (5H, m,Ph) 7.72 and 8.22 (each 2H, d, J 9Hz, $C_6H_4-NO_2$).

Example 2b

Sodium (5R,6R)-3-[(Z)-2-benzylaminocarbonylethenylthio]-6-[(S)-1-hydroxyethyl]-7-oxo-1-azabicyclo[3.2.0]hept-2-ene-2-carboxylate

(e7)

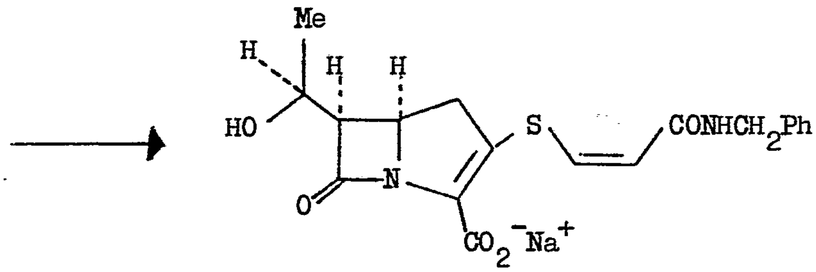

(e8)

The ester (e7) (10 mg) and 5% Pd-C catalyst (15 mg) were shaken under hydrogen in a mixture of dioxan (5 ml), water (2 ml) and 0.05 M pH 7 phosphate buffer (0.5 ml). After 2.25 h, the mixture was filtered, over Celite, washing the pad with water (20 ml). After the addition of $NaHCO_3$ (3 mg), the filtrate was concentrated in vacuo to a volume of ca. 20 ml, and then washed with ethyl acetate (3 x 20 ml). The aqueous solution was further concentrated in vacuo to afford a solution of the title sodium salt (e8); $\lambda_{max.}$ ($H_2O$) 322 nm.

Example 3a

p-Nitrobenzyl (5R,6S)-3-[(Z)-2-benzylaminocarbonylethenylthio]-6-[(R)-1-formyloxyethyl]-7-oxo-1-azabicyclo[3.2.0]hept-2-ene-2-carboxylate

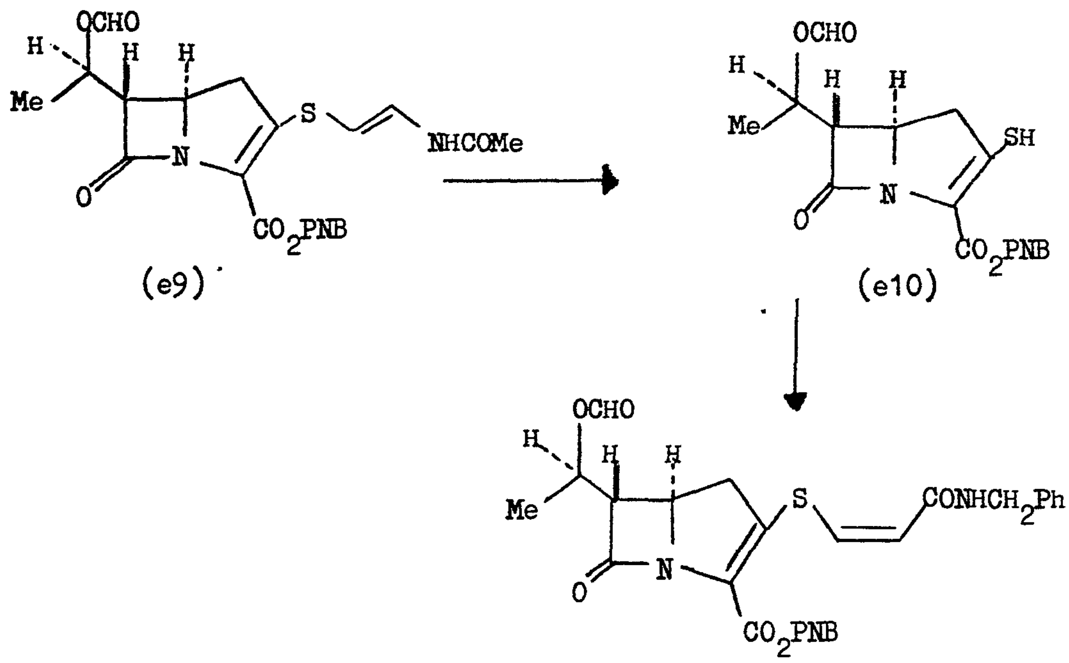

(e9) (e10)

(e11)

A The ester (e9) (200 mg) was treated with N-bromoacetamide (58 mg) using the method described in Example 1a (A) to afford a product which contained the thiol (e10).

B The product above was stirred with N-benzylpropiolamide (e6) (133 mg) and potassium carbonate (30 mg) in N,N-dimethylformamide for 20 min. Work-up as in Example 1a (B) followed by chromatography on silica gel, using a gradient elution of 50% ethyl acetate-hexane to 100% ethyl acetate, afforded the title product (e11) as a gum (20 mg); $\lambda_{max.}$ (EtOH) 335 and 263 nm; $\nu_{max.}$ ($CH_2Cl_2$) 3340, 1790, 1730, 1705 sh and 1660 $cm^{-1}$; $\delta$ ($CDCl_3$) 1.42 (3H, d, J 6.5 Hz, MeCH), ca. 3.3 (3H, m, 4-$CH_2$ and 6-CH), 4.22 (1H, m, 5-CH),

4.47 (2H, d, J 6Hz, $CH_2Ph$), ca. 5.35 (3H, m, CHMe and $CH_2Ar$), 5.90 (1H, d, J 10 Hz, CO.CH=), ca. 7.1 (1H, d, J 10 Hz, = CH.S), 7.55 br (1H, NH), 8.02 (1H, s, OCHO), 7,65 and 8.20 (each 2H, d, J 9Hz, $C_6H_4$-$NO_2$).

Example 3b

Sodium (5R,6S)-3-[(Z)-2-benzylaminocarbonylethenylthio]-6-[(R)-1-formyloxyethyl]-7-oxo-1-azabicyclo[3.2.0]hept-2-ene-2-carboxylate

(e11)

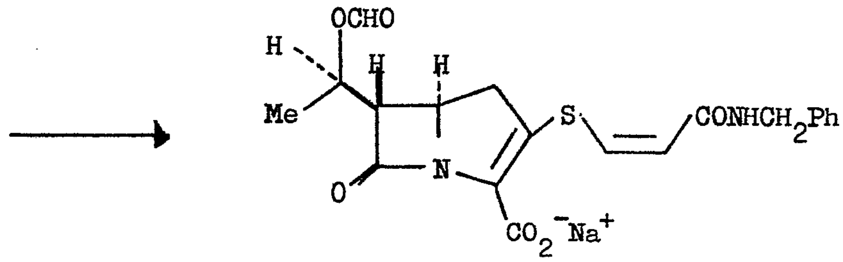

(e12)

The ester (e11) (20 mg) was hydrogenolysed by the method described in Example 2b. Work-up employing sodium bicarbonate (5 mg) afforded an aqueous solution of the title salt (e12); $\lambda_{max.}$ ($H_2O$) 318 nm.

Example 4

Sodium (5R,6S)-3-[(Z)-2-benzylaminocarbonylethenylthio]-6-[(R)-1-hydroxyethyl]-7-oxo-1-azabicyclo[3.2.0]hept-2-ene-2-carboxylate.

(e12)

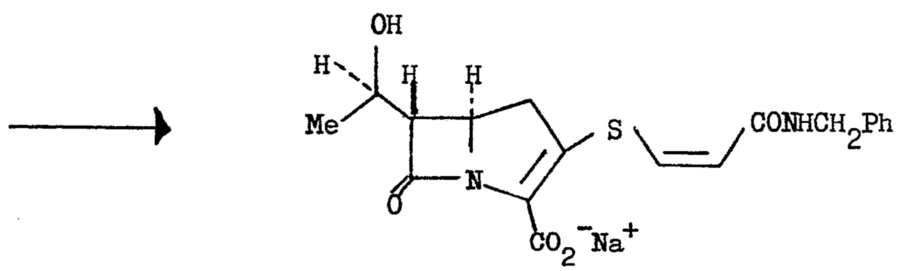

(e13)

An aqueous solution of the formate (e12) (45 mg in 10 ml) was adjusted to pH 9 using 0.1 N sodium hydroxide solution. The solution was maintained at pH 9 for 2 h at room temperature and the pH then allowed to fall to 8.5. The solution was then kept at 5° for 16 h and the pH then adjusted to 9 for a further 15 min. H.p.l.c.

(20% acetonitrile in pH 4.7 ammonium phosphate buffer solution) showed that hydrolysis of the formate ester was virtually complete. The solution was then neutralised to pH 7 with 0.1 N aqueous HCl and concentrated to a volume of 5 ml before loading onto a column of Diaion HP20. Elution with 40% ethanol-water afforded a solution of the title sodium salt (e13); $\lambda_{max.}$ ($H_2O$) 322 nm.

Example 5a

2-Carbamoylethenylthioacetate (e14) and (e15)

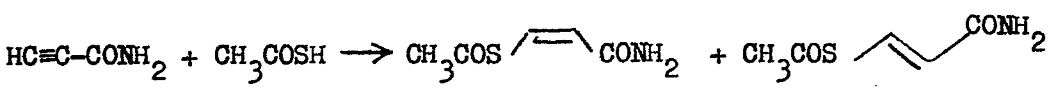

(e14) (e15)

Propiolamide (640 mgs) in dry dimethylformamide (DMF), (5 ml) was treated with potassium carbonate (13.8 mgs) and thiolacetic acid (705 mgs) with cooling. After 30 mins t.l.c. analysis (ethyl acetate) showed absence of starting material. The solution was diluted with ethyl acetate (50 ml) and washed with brine (3 x 50 ml). The organic layer was dried and the solvent removed to give a brown semi-solid product. This was chromatographed on silica gel 60 (Merck Art 7729; 230 - 400 ASTM) eluting with ethyl acetate:hexane, 1:1. The first product, on trituration with ether, gave Z-2-carbamoylethenylthioacetate (e14) as a colourless solid, 207 mg (15%). $\nu_{max.}$ ($CHCl_3$) 3525, 3410, 1705, 1680, and 1590 $cm^{-1}$. $\delta$ (d6-acetone) 2.43 (3H, s, $C\underline{H}_3CO-$), 6.29 (1H, d, $\underline{J}$ 10Hz; $COC\underline{H}=$), 7.55 (1H, d, $\underline{J}$ 10Hz; $=C\underline{H}.S$), ~6.1 - 7.8 (2H, v. broad s, $-\underline{NH}_2$).

The second component was E-2-carbamoylethenylthioacetate (e15), isolated as a solid, 130 mgs, (10%). $\nu_{max.}$ 3525, 3410, 1710, 1680 and 1590 $cm^{-1}$ $\delta$ (d6-acetone) 2.47 (3H, s, $C\underline{H}_3CO-$), 6.35 (1H, d, $\underline{J}$ 16Hz; $-COCH=$), 7.94 (1H, d, $\underline{J}$ 16Hz; = $C\underline{H}$ S-), ~6.1 - 7.5 (2H, v. broad s, $-\underline{NH}_2$).

Example 5b

p-Nitrobenzyl 3-[E-2-carbamoylethenylthio]-6-[1-p-nitrobenzyloxycarbonyloxyethyl]-7-oxo-1-azabicyclo[3.2.0]hept-2-ene-2-carboxylate (e 17)

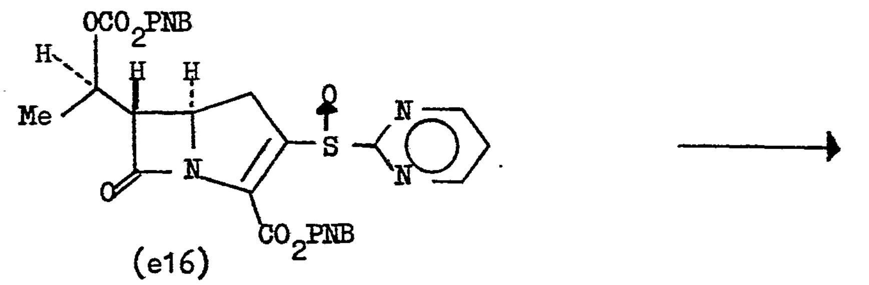

(e16)

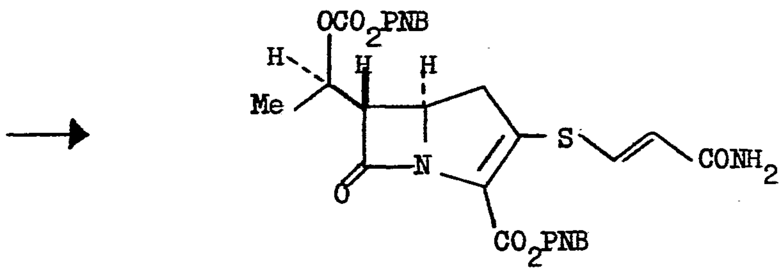

(e17)

E-2-Carbamoylethenylthioacetate (e15), (23 mgs) in dioxan (3ml) was treated with an aqueous solution of 0.1 M sodium hydroxide (3.2 ml). After 15 min. at ambient temperature, the dioxan was removed in vacuo and a further quantity of water (3 ml) was added.

This solution was added to a solution of p-nitrobenzyl 3-[pyrimidin-2-ylsulphinyl]-6-[1-p-nitrobenzyloxycarbonyloxyethyl]-7-oxo-1-azabicyclo[3.2.0]hept-2-ene-2-carboxylate (e16), (100 mg) in dichloromethane (6 ml), at $0^{o}C$. Cetyldimethylbenzylammonium chloride (1 mg) was added and the mixture vigorously stirred. After ca. 12 hrs t.l.c. analysis (ethyl acetate:ethanol, 9:1) showed reaction had proceeded to completion. The mixture was diluted with dichloromethane and the organic phase separated, dried and the solvent removed to give an off-white solid upon trituration with ether. Chromatography on silica gel 60 (Merck Art. 7729; 230 - 400 ASTM) eluting with ethyl

acetate:hexane; 3:1, gave p-nitrobenzyl 3-[E-2-carbamoylethenylthio]-6-[1-p-nitrobenzyloxycarbonyloxyethyl]-7-oxo-1-azabicyclo[3.2.0]-hept-2-ene-2-carboxylate (e17), (15 mgs; 15%) as a white solid. $\nu_{max.}$ ($CHCl_3$), 1790, 1745, 1690(b), 1610, 1590, 1560, 1525 and 1350 $cm^{-1}$; δ (d6-acetone), 1.45 (3H, d, J 6Hz; $-C\underline{H}_3$), 3.48 (1H, dd, J 8, 18Hz; C4-H), 3.59 (1H, dd, J 10, 18 Hz; C4-H), 3.83 (1H, dd, J 3, 6 Hz; C6-H), 4.45 (1H, m, C5-H), 5.22 (1H, dt, J 7, 14 Hz; C8-H), 5.35 (2H, s, $-OCO_2C\underline{H}_2Ph$), 5.55 (2H, ABq, $-CO_2C\underline{H}_2Ph$), 6.38 (1H, d, J 17 Hz; = $C\underline{H}.CONH_2$), 6.46 (1H, broad s, -N$\underline{H}$-), 7.00 (1H, broad s, -N$\underline{H}$-), 7.65 (1H, d, J 17 Hz; -SC$\underline{H}$=), 7.68 and 7.78 (4H, d, J 9 Hz; arom -$\underline{H}$), 8.25 and 8.26 (4H, d, J 9Hz; arom -$\underline{H}$).

Example 5c

Sodium 3-[E-2-carbamoylethenylthio]-6-[1-hydroxyethyl]-7-oxo-1-azabicyclo[3.2.0]hept-2-ene-2-carboxylate (e19)

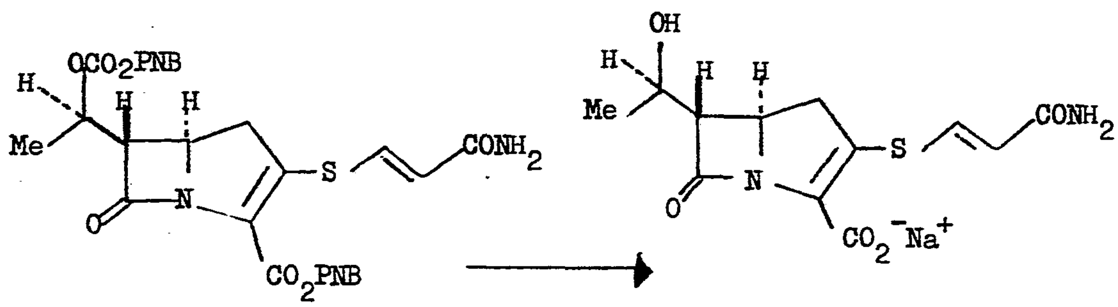

(e17) (e19)

10% Palladium/charcoal catalyst (15 mg), suspended in 50% aqueous dioxan (4 ml) was pre-hydrogenolysed for 20 mins. To this was then added a solution of p-nitrobenzyl 3-[E-2-carbamoylethenyl thio]-6-[1-p-nitrobenzyloxycarbonyloxyethyl]-7-oxo-1-azabicyclo [3.2.0]hept-2-ene-2-carboxylate (e17), (11 mg) in 50% aqueous dioxan (6 ml). After hydrogenolysis for 2½ hr., sodium hydrogen carbonate (1.5 mg) in water (1 ml) was added. The dioxan was removed in vacuo and the aqueous phase extracted with ethyl acetate. The aqueous solution was shown to contain 3.75 mgs; (65%), of sodium 3-[E-2-carbamoylethenylthio]-6-[1-hydroxyethyl]-7-oxo-1-azabicyclo[3.2.0] hept-2-ene-2-carboxylate (e19), $\lambda_{max.}$ ($H_2O$) 316 nm.

Example 6a

p-Nitrobenzyl 3-[Z-2-carbamoylethenylthio]-6-[1-p-nitrobenzyloxy carbonyloxyethyl]-7-oxo-1-azabicyclo[3.2.0]hept-2-ene-2-carboxylate (e18)

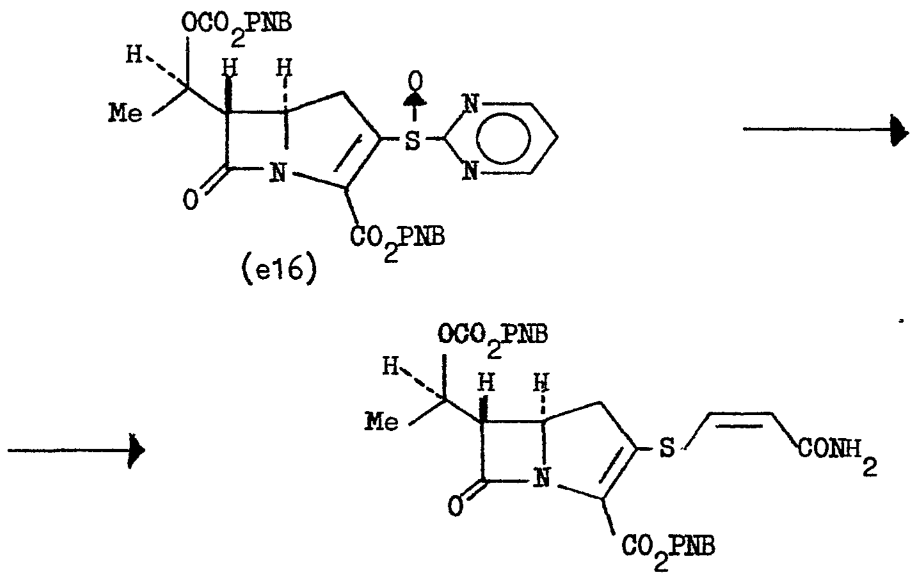

(e16)

(e18)

Z-2-Carbamoylethenylthioacetate (e14), (23 mgs) in dioxan (3 ml) was treated with an aqueous solution of 0.1 M sodium hydroxide (3.2 ml). After 30 mins at ambient temperature the dioxan was removed in vacuo and a further 3 ml of water added.

This solution was added to a solution of p-nitrobenzyl-3-[pyrimidin-2-ylsulphinyl]-6-[1-p-nitrobenzyloxycarbonyloxyethyl]-7-oxo-1-azabicyclo[3.2.0]hept-2-ene-2-carboxylate (e16), (100 mg) in dichloromethane (6 ml) at 0°C. Cetyldimethylbenzylammonium chloride (1 mg) was added. After stirring vigorously for 30 mins at 0°C and ca 12 hrs at ambient temperature, t.l.c. analysis (ethyl acetate) showed completion of reaction. The phases were partitioned and the aqueous phase extracted with dichloromethane. The organic phases were combined, dried and the solvent removed. Chromatography on silica gel 60 (Merck Art. 7729; 230 - 400 ASTM) eluting with ethyl acetate, gave a yellow solid, which, upon trituration with acetone/ether gave a

white solid shown to be p-nitrobenzyl 3-[Z-2-carbamoylethenylthio]-6-[1-p-nitrobenzyloxycarbonyloxyethyl]-7-oxo-1-azabicyclo[3.2.0]hept-2-ene-2-carboxylate (e18), (15 mgs; 15%). $\nu_{max.}$ 1795, 1745, 1690(b), 1605, 1590, 1560, 1525 and 1350 $cm^{-1}$; δ (d6-DMSO) 1.36 (3H, d, J 7 Hz; $-C\underline{H}_3$), 3.41 - 3.62 (m, 2CH-H masked by $H_2O$), 3.89 (1H, dd, J 3, 6Hz; C6-H), 4.29 (1H, m, C5-H), 5.00 (1H, quint., J 6Hz; C8-H), 5.33 (2H, s, $-OCO_2C\underline{H}_2Ph$), 5.44 (2H, ABq, $-CO_2C\underline{H}_2Ph$), 6.58 (1H, d, J 10Hz; = C$\underline{H}$CO-), 7.65 and 7.76 (4H, d, J 9Hz; arom -$\underline{H}$), 7.72 (1H, d, J 10Hz; -SC$\underline{H}$=), 8.24 (4H, d, J 9Hz; arom-H).

Example 6 b

Sodium 3-[Z-2-carbamoylethenylthio]-6-[1-hydroxyethyl]-7-oxo-1-azabicyclo[3.2.0]hept-2-ene-2-carboxylate (e20)

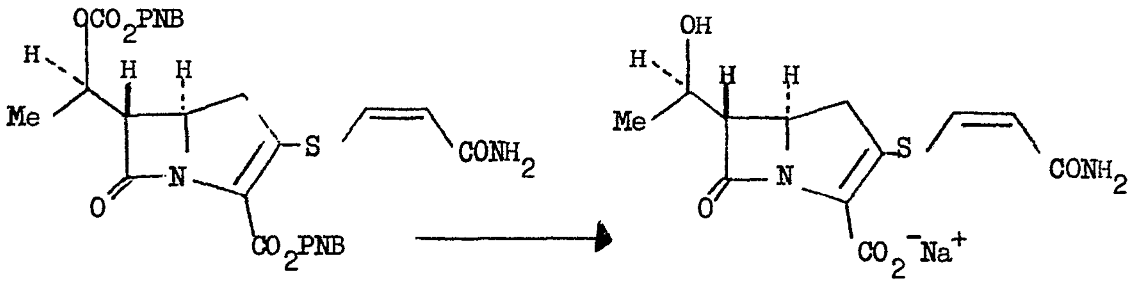

(e18) (e20)

10% Palladium/charcoal catalyst (10 mg) in 50% aqueous dioxan (4 ml) was pre-hydrogenolysed for 20 min. To this was added a solution of p-nitrobenzyl 3-[Z-2-carbamoylethenylthio]-6-[1-p-nitrobenzyloxycarbonyloxyethyl]-7-oxo-1-azabicyclo[3.2.0]hept-2-ene-2-carboxylate (e18), (10 mg) in 50% aqueous dioxan (10 ml). After hydrogenolysis for 2½ hr., sodium hydrogen carbonate (1.4 mg) in water (1 ml) was added. The dioxan was removed in vacuo. The aqueous solution was extracted with ethyl acetate to give an aqueous solution of 3.5 mgs (67%) of sodium 3-[Z-2-carbamoylethenylthio]-6-[1-hydroxyethyl]-7-oxo-1-azabicyclo[3.2.0]hept-2-ene-2-carboxylate (e20), $\lambda_{max.}$ ($H_2O$) 338 nm.

Example 7a

Z-3-acetylthioprop-2-enoic acid

Propiolic acid (10 g) in dioxan (100 ml) at $0^{o}C$, was treated with 0.1 M aqueous sodium hydroxide (142 ml). After 5 minutes, thiolacetic acid (10.9 g) was added. After 3 hours, the dioxan was removed and the aqueous phase extracted with ethyl acetate (3 x 100 ml portions). Combination of organic phases, drying and removal of solvent *in vacuo* afforded the crude pale yellow product. Chromatography on silica gel 60 (Merck Art. 7729; <230 ASTM) eluting with ethyl acetate:hexane, 7:10 followed by recrystallization from ethyl acetate/hexane gave Z-3-acetylthioprop-2-enoic acid, 5.98 g (29%). $\nu_{max.}$ ($CHCl_3$) 3030 (v.b.), 1710, 1690 and 1595$cm^{-1}$; $\delta$ ($CDCl_3$) 2.53 (3H, s, $C\underline{H}_3COS-$), 6.17 (1H, d, J10Hz; $-COC\underline{H}=$), 8.00 (1H, d, J10Hz; $-SC\underline{H}=$), 11.11 (1H, broad s, $-O\underline{H}$).

Example 7b

N-methylcarbamoylethenylthioacetate

Z-3-Acetylthioprop-2-enoic acid, (500 mg) was dissolved in dry benzene (40 ml) and treated with oxalyl chloride (870 mg) at room temperature for 48 hours. The solvent was removed and replaced with dry dichloromethane (10 ml). The solution was cooled to 0°C and treated with 1.22 mls of a solution of anhydrous methylamine (2.17 g) in dry dichloromethane (25 ml) in a dropwise fashion followed by triethylamine (0.476 ml). After 30 minutes, t.l.c. analysis showed the presence of two products. These were separated by chromatography on silica gel 60 (Merck Art. 7729; <230 ASTM) eluting with ethyl acetate:hexane, 1:1.

Z-(N-methylcarbamoyl)ethenylthioacetate was eluted first as a white solid; 356 mg (65%), recrystallised from ethyl acetate/hexane, m.p. 106-114°C; $\nu_{max.}$ ($CHCl_3$) 3450, 3325 (b), 1700, 1640, 1600 (m) and 1520cm$^{-1}$; $\delta$($CDCl_3$) 2.46 (3H, s, $C\underline{H}_3CO-$) 3.94 (3H, d, J5Hz; $-NC\underline{H}_3$), 6.15 (1H, d, J10Hz; $-COC\underline{H}=$), 6.37 (1H, broad s, $-N\underline{H}-$), 7.64 (1H, d, J10Hz; $-SC\underline{H}=$).

The second component was E-(N-methylcarbamoyl)ethenylthioacetate eluted as a white solid; 70 mg (13%) recrystallised from ethyl acetate, m.p. 156-160°C; $\nu_{max.}$ ($CHCl_3$) 3450, 3375 (v.b.) 1710, 1660, 1595 and 1320cm$^{-1}$; $\delta$($CDCl_3$) 2.42 (3H, s, $C\underline{H}_3CO-$), 2.95 (3H, d, J5Hz; $-NC\underline{H}_3$), 5.70 (1H, broad s, $-N\underline{H}-$), 6.17 (1H, d, J16Hz; $COC\underline{H}=$), 7.97 (1H, d, J16Hz; $-SC\underline{H}=$).

Example 7c

p-Nitrobenzyl 3-[E-2-(N-methylcarbamoyl)ethenylthio]-6-[1-p-nitrobenzyloxycarbonyloxyethyl]-7-oxo-1-azabicyclo[3.2.0]hept-2-ene-2-carboxylate.

a). E-(N-methylcarbamoyl)ethyenylthioacetate (36 mg) was dissolved in dioxan (4 ml) and treated with 0.1 M aqueous sodium hydroxide (4.1 ml). After 15 minutes the dioxan was removed in vacuo to give a yellow aqueous solution of sodium E-(N-methylcarbamoyl)ethenylthiolate.

b). p-Nitrobenzyl-3-[pyrimidin-2-ylsulphinyl]-6-[1-p-nitrobenzyloxycarbonyloxyethyl]-7-oxo-1-azabicyclo[3.2.0]hept-2-ene-2-carboxylate (128 mg) in dichloromethane (8 ml) was cooled to 0°C. Cetyldimethylbenzylammonium chloride (1 mg) was added followed by the previously prepared sodium thiolate with vigorous stirring. After 3 hours the phases were separated. The aqueous phase was extracted with ethyl acetate (2 x 25 ml). The organic phases were combined, dried and the solvent removed. Column chromatography on silica gel 60 (Merck Art. 7729; <230 ASTM) eluting with ethyl acetate afforded a yellow foam. Trituration with ether afforded the title compound as a pale yellow solid, 52 mg (41%) $\nu_{max.}$ ($CHCl_3$) 3450 (w), 1790, 1750, 1705 (w), 1660, 1610 (w), 1590, 1560, 1525, 1380 and 1350 $cm^{-1}$; $\lambda_{max.}$ (Ethanol) 262 nm (ε, 24,570), 331 nm (ε, 24,840); δ($CDCl_3$) 1.49 (3H, d, J7Hz; $-C\underline{H}_3$), 2.90 (3H, d, J5Hz; $CON-C\underline{H}_3$), 3.22 (1H, dd, J8, 18Hz; C4-H), 3.44 (1H, dd, J3,8Hz; C6-H), 3.48 (1H, dd, J10, 18Hz; C4-H), 4.32 (1H, m, C5-H), 5.18 (1H, m, C8-H), 5.26 (2H, s, $-CO_2C\underline{H}_2Ph$), 5.39 (2H, ABq, J14Hz; $-OCO_2C\underline{H}_2Ph$), 6.10 (1H, d, J15Hz; $-SC\underline{H}=$), 7.56 and 7.64 (4H, 2d, J9Hz; arom-H), 7.65 (1H, d, J15Hz; $-NCOC\underline{H}=$), 8.22 and 8.26 (4H, 2d, J9Hz; arom-H); m/e 429(5) and 361(8%).

Example 7d

Sodium 3-[E-2-(N-methylcarbamoyl)ethenylthio]-6-[1-hydroxyethyl]-7-oxo-1-azabicyclo[3.2.0]hept-2-ene-2-carboxylate

10% Palladium/charcoal catalyst (15 mg) as a suspension in 50% aqueous dioxan (4 ml) was pre-hydrogenolysed for 20 minutes. To this was added p-nitrobenzyl 3-[E-2-(N-methylcarbamoyl)ethenylthio]-6-[p-nitrobenzyloxycarbonyl-oxyethyl]-7-oxo-1-azabicyclo[3.2.0]hept-2-ene-2-carboxylate (15 mg) in 50% aqueous dioxan (6 ml). The total mixture was hydrogenolysed for 2.5 hours. Then sodium hydrogen carbonate (2.01 mg) in water (1 ml) was added. The mixture was filtered through celite and the dioxan evaporated under vacuum. The aqueous solution was extracted with ethyl acetate (3 x 20 ml) to give an aqueous solution containing 6.85 mgs (86%) of the title compound. $\lambda_{max.}$ ($H_2O$) 318 nm.

Example 8a

p-Nitrobenzyl 3-[E-2-(N-acetyl-N-methylcarbamoyl)ethenylthio[-6-[1-p-nitrobenzyloxycarbonyloxyethyl]-7-oxo-1-azabicyclo[3.2.0]hept-2-ene-2-carboxylate

a). Z-(N-methylcarbamoyl)ethenylthioacetate (25.4 mg) in dioxan (3 ml) was treated with 0.1 M aqueous sodium hydroxide (3.2 ml). After 15 minutes the dioxan was removed to give a yellow aqueous solution.

b). p-Nitrobenzyl-3-[pyrimidin-2-ylsulphinyl]-6-[1-p-nitrobenzyloxycarbonyloxyethyl]-7-oxo-1-azabicyclo[3.2.0]hept-2-ene-2-carboxylate (100 mg) in dichloromethane (6 ml) was cooled to 0°C. Cetyldimethylbenzylammonium chloride (1 mg) was added followed by the previously prepared aqueous solution.

The mixture was allowed to stir vigorously at room temperature for 2 hours. T.l.c. (ethyl acetate) analysis showed completion of reaction, with the formation of one major product. The organic phase was separated and the aqueous phass extracted with dichloromethane. Combination, drying and removal of solvent from the organic phases afforded a yellow gum. This was subjected to column chromatography on silica gel 60 (Merck Art. 7729; <230 ASTM) eluting with ethyl acetate:hexane, 7:10. The title compound was obtained as a pale yellow solid, after trituration with ether. Yield 30 mgs (30%). m.p. 139-140°C (ex. ethyl acetate/hexane); $\nu_{max.}$ ($CHCl_3$) 1790, 1730, 1710, 1610 (w), 1555, 1525 and 1480$cm^{-1}$; $\lambda_{max.}$ (ethanol) 260 nm (ε, 20,550), 343 nm (ε, 20,380); δ ($CDCl_3$) 1.50 (3H, d, J7Hz; C-$C\underline{H}_3$), 2.42 (3H, s, $CONC\underline{H}_3$), 3.26 (3H, s, -$COC\underline{H}_3$ and 1H, m, C4-H), 3.44-3.54 (2H, m, C4-H and C6-H), 4.33 (1H, m, C5-H; $J_{5-6}$ 13.13Hz), 5.18 (1H, m, C8-H; $J_{6-8}$ 7.80Hz), 5.27 (2H, s, -$CO_2C\underline{H}_2Ph$), 5.40 (2H, ABq, J13Hz; -$OCO_2C\underline{H}_2Ph$), 6.94 (1H, d, J14.9Hz; -$SC\underline{H}$=), 7.56 and 7.65 (4H, 2d, J9Hz; arom -H), 7.80 (1H, d, J14.9Hz; -$NCOC\underline{H}$=), 8.24 and 8.26 (4H, 2d, J9Hz; arom - H); m/e 404(5), 331(3) and 285(17%); Found C, 54.32; H, 4.45; N, 8.40; $C_{30}H_{28}N_4O_{12}S$ requires C, 53.89; H, 4.22; N, 8.38%.

Example 8b

Sodium 3-[E-2-(N-acetyl-N-methylcarbamoyl)ethenylthio]-6-[1-hydroxyethyl]-7-oxo-1-azabicyclo[3.2.0]hept-2-ene-2-carboxylate

10% Palladium/charcoal catalyst (15 mg) suspended in 50% aqueous dioxan (4 ml) was prehydrogenolysed for 20 minutes. p-Nitrobenzyl 3-[E-2-(N-acetyl-N-methylcarbamoyl)ethenylthio]-6-[1-p-nitrobenzyloxycarbonyloxyethyl]-7-oxo-1-azabicyclo[3.2.0]hept-2-ene-2-carboxylate (15 mgs) in aqueous dioxan (6 ml) was added and the total mixture hydrogenolysed for a further 2.5 hours. Sodium hydrogen carbonate (2 mgs) was added and the mixture filtered through celite. Removal of dioxan, followed by extraction of the aqueous solution with ethyl acetate (2 x 20 ml) afforded a solution containing 9.7 mgs of the title compound (65%) $\lambda_{max.}$ ($H_2O$) 340 nm.

Example 9a

Z-(N-2-Bromoethylcarbamoyl)ethenylthioacetate

3-Acetylthioprop-2-enoic acid (1 g) in dry benzene (80 ml) was treated with oxalyl chloride (1200 µl) at room temperature for 24 hours. Solvent removed was replaced with dry dichloromethane (10 ml) and the solution cooled to $0^{o}C$. To this was added a solution of 2-bromoethylamine hydrobromide (1.404 g) in dichloromethane (10 ml) followed by triethylamine (1.904 ml). After 15 minutes the solution was washed with brine, dried and the solvent removed. The solid crude product was recrystallised from ethyl acetate/hexane to give pure Z-(N-2-bromoethylcarbamoyl)ethenylthioacetate, 1.087 g (63%); m.p. 102.5-104$^{o}$C; $\nu_{max.}$ ($CHCl_3$) 3440(w), 1705, 1665 (m.w.) and 15.0cm$^{-1}$; δ ($CDCl_3$) 2.46 (3H, s, $C\underline{H}_3COS-$), 3.67 (4H, m, $-(C\underline{H}_2)_2-$), 6.05 (2H, d, J10Hz; $-COC\underline{H}=$, broad s, $-N\underline{H}-$), 7.70 (1H, d, J10Hz; $-SC\underline{H}=$); m/e Found 250.9617, $C_7H_{10}NO_2SBr$ requires 250.9613; 211(18), 210(15), 209(18), 208(13), 172(9), 130(65) and 128(30%); Found: C, 34.06; H, 4.07; N, 5.51; S, 13.04; Br, 31.74: $C_7H_{10}BrNO_2S$ requires C, 33.34; H, 4.00; N, 5.56; S, 12.72; Br, 31.69%.

Example 9c

E-(N-2-bromoethylcarbamoyl)ethenylthioacetate

Z-(N-2-bromoethylcarbamoyl)ethenylthioacetate (200 mgs) in dry benzene (10 ml) and a suspension of 5% palladium/alumina (20 mgs) were heated under reflux for about 2 hours. T.l.c. analysis showed presence of new product with unchanged starting material. The solution was allowed to cool and filtered through celite. The residue obtained after removal of solvent was chromatographed on silica gel 60 (Merck Art. 7729; <230 ASTM) eluting with ethyl acetate:hexane, 1:1. The first compound eluted was recovered starting material obtained as a white crystalline solid (47%). The product was eluted as the second component and shown to be the title trans isomer (45 mgs, 23%). $\nu_{max.}$ ($CHCl_3$) 3430, 1710, 1660, 1595 and 1510cm$^{-1}$; δ($CDCl_3$) 2.43 (3H, s, $C\underline{H}_3CO-$), 3.37-3.95 (4H, m, $-(C\underline{H}_2)_2-$), 6.20 (1H, d, J16Hz; $-COC\underline{H}=$), 6.56 (1H, broad s, $-N\underline{H}-$), 8.02 (1H, d, J16Hz; $-SC\underline{H}=$).

Example 9c

p-Nitrobenzyl 3-(E-2-(N-2-bromoethylcarbamoyl)ethenylthio]-6-[1-p-nitrobenzyloxycarbonyloxyethyl]-7-oxo-1-azabicyclo[3.2.0]hept-2-ene-2-carboxylate

a). E-(N-2-bromoethylcarbamoyl)ethenylthioacetate (30 mg) in dioxan (3 ml) was treated with 0.1 M aqueous sodium hydroxide (3.77 ml) at room temperature for 10 minutes. The dioxan was evaporated under vacuum to give a yellow aqueous solution.

b). p-Nitrobenzyl-3-[pyrimidin-2-ylsulphinyl]-6-[1-p-nitrobenzyloxycarbonyloxyethyl]-7-oxo-1-azabicyclo[3.2.0]hept-2-ene-2-carboxylate (120 mg) in dichloromethane (8 ml) and cetyldimethylbenzylammonium chloride (2 mg) was cooled to $0^{o}C$. The aqueous solution from a) was added to this and the mixture vigorously stirred at room temperature for 3 hours.

The phases were separated and the organic phase washed with water. The combined aqueous phases were extracted with dichloromethane (10 ml). The combined organic phases were dried and the solvent removed. The title product was eventually obtained after silica gel 60 (Merck Art. 7729; <230 ASTM) chromatography eluting with ethyl acetate, as a pale yellow solid, 36 mgs (27%). $\nu_{max.}$ ($CHCl_3$) 3380 (w,b) 1790, 1750, 1705(w), 1645, 1610, 1525(s), 1445, 1385 and 1350$cm^{-1}$; $\lambda_{max.}$ ($CH_3CN$) 264 nm ($\epsilon$, 27,650), 331nm ($\epsilon$, 26,570); $\delta$($CDCl_3$) 1.49 (3H, d, J7Hz; $-C\underline{H}_3$), 3.19 (1H, dd, J8.5, 18Hz; C4-H), 3.42 (1H, dd, J10, 18Hz; C4-H), 3.43 (1H, dd, J3,8Hz; C6-H), 3.96 (2H, t, J9.5Hz; $-C\underline{H}_2-$), 4.29 (3H, t, J9.5Hz; $-C\underline{H}_2-$ and m, C5-H), 5.17 (1H, m, C8-H), 5.27 (2H, s, $-CO_2C\underline{H}_2Ph$), 5.40 (2H, ABq, J14Hz; $-OCO_2C\underline{H}_2Ph$), 6.36 (1H, d, J15.7Hz; $-SC\underline{H}=$), 7.29 (1H, d, J15.7Hz; $=C\underline{H}CON-$), 7.56 and 7.64 (4H, 2d, J9Hz; arom -H), 8.22 and 8.25 (4H, 2d, J9Hz; arom -H).

Example 9d

Sodium 3-[E-2-(N-2-bromoethylcarbamoyl)ethenylthio]-6-[1-hydroxyethyl]-7-oxo-1-azabicyclo[3.2.0]hept-2-ene-2-carboxylate

10% Palladium/charcoal (10 mg) as a suspension in 50% aqueous dioxan (4 ml) was pre-hydrogenolysed for 20 minutes. To this was added a solution of p-nitrobenzyl 3-[E-2-(N-2-bromoethylcarbamoyl)ethenylthio]-6-[1-p-nitrobenzyloxycarbonyloxyethyl]-7-oxo-1-azabicyclo[3.2.0]hept-2-ene-2-carboxylate (10 mg) in 50% aqueous dioxan (8 ml) and the mixture hydrogenolysed for 3 hours. 0.1M aqueous sodium hydrogen carbonate (143 µl) was added, and the reaction mixture was filtered through celite. The dioxan was removed under vacuum and the aqueous solution extracted with ethyl acetate (3 x 20 ml).

The aqueous solution contained 3.47 mg (61%) of the title compound. $\lambda_{max.}$ ($H_2O$) 319 nm.

Example 10a

2-p-Nitrobenzyloxycarbonylaminoethanol

Ethanolamine (1 g) in dichloromethane (20 ml) was cooled to 0°C and triethylamine (1.66 g) was added. Then a solution of p-nitrobenzylchloroformate (3.53 g) in dichloromethane (20 ml) was added in a dropwise fashion. After 3 hours at room temperature the solution was washed with brine. After drying, the solvent was removed to give a pale brown crystalline solid. 2.16 g (55%) m.p. (ex. ethyl acetate) 101.5°C; $\nu_{max.}$ ($CHCl_3$), 3450(s), 1720, 1610(w), 1515 and 1350cm$^{-1}$; δ($CDCl_3$) 2.63 (1H, broad s, exchanges with $D_2O$, -OH), 3.41 (2H, t, J6Hz; $-CH_2N-$), 3.75 (2H, t, J6Hz; $-CH_2O-$), 5.23 (2H, s, $-CH_2Ph$), 5.49 (1H, broad s, -NH-), 7.50 and 8.23 (4H, 2d, J9Hz; arom.-H); Found: C, 50.06; H, 5.10; N, 11.58: $C_{10}H_{12}N_2O_5$ requires C, 50.00; H, 5.04; N, 11.58%.

Example 10b

2-p-Nitrobenzyloxycarbonylaminoethylazide

2-p-Nitrobenzyloxycarbonylaminoethanol (300 mg) in dry tetrahydrofuran (50 ml) was cooled to 0°C. Triphenylphosphine (655 mg) and a 1.7 M toluene solution of hydrazoic acid (1.47 ml) were added. After the addition of diisopropylazodicarboxylate (505 mg) the reaction mixture was stirred for 10 minutes. The solvent was removed in vacuo, and the residue taken up in ethyl acetate, washed with brine and dried. After removal of solvent the crystalline residue was chromatographed on silica gel 60 (Merck Art. 7729; <230 ASTM) eluting with dichloromethane to give the title compound as a crystalline solid. 315 mg (95%), m.p. (ex. ethyl acetate/hexane) 57°C; $\nu_{max.}$ ($CHCl_3$) 3450, 2110 (s), 1720(s), 1610(w), 1510, 1450 and 1350cm$^{-1}$; δ ($CDCl_3$) 3.46 (4H, broad s, $-(CH_2)_2-$), 5.23 (2H, s, $-CH_2Ph$), 5.40 (1H, v. broad s, -NH-), 7.49 and 8.19 (4H, 2d, J9Hz; arom -H); Found: C, 45.14; H, 4.14; N, 26.62; $C_{10}H_{11}N_5O_4$ requires C, 45.28; H, 4.18; N, 26.41%; m/e 283($MNH_4^+$) (15), 267 (15), 266 ($MH^+$) (100), 238 (5), 209 (4), 194 (5), 185 (55), 153 (44), 151 (5) and 136 (94%).

Example 10c

Z-(N-2-p-nitrobenzyloxycarbonylaminoethylcarbamoyl)ethenylthioacetate.

2-p-Nitrobenzyloxycarbonylaminoethylazide (100 mg) was dissolved in dry toluene (10 ml) and heated to 65°C in the presence of triphenylphosphine (99 mg). T.l.c. analysis showed no starting material after 2 hours. The solution was allowed to cool, and then further cooled to 0°C. 2-Acetylthioprop-2-enoyl chloride (62 mg) in toluene (2 ml) was added, whereupon an immediate white precipitate formed. After 15 minutes ethyl acetate (10 ml) and aqueous saturated sodium hydrogen carbonate (10 ml) were added. The organic phase was separated and washed with brine. The combined aqueous phases were extracted with ethyl acetate. The organic phases were combined, dried and the solvent removed. Silica gel 60 (Merck Art. 7729; <230 ASTM) chromatography, eluting with ethyl acetate:hexane, 7:10, followed by recrystallisation from ethyl acetate/hexane, afforded the title compound 32 mg (21%), m.p. 116.5-118°C ; $\nu_{max.}$ ($CHCl_3$) 3500-3200 (v b), 1710, 1655, 1610(w), 1595, 1515 and 1350cm$^{-1}$; $\delta$($CDCl_3$) 2.41 (3H, s, $C\underline{H}_3CO-$), 3.41 (4H, broad s, $-(C\underline{H}_2)_2-$), 5.16 (2H, s, $-C\underline{H}_2Ph$), 5.43 (1H, broad s, $-N\underline{H}CO_2-$), 5.96 (1H, d, J10Hz; $-COC\underline{H}=$), 6.26 (1H, broad s, $-N\underline{H}CO-$), 7.45 and 8.17 (4H, 2d, J9Hz; arom -H), 7.60 (1H, d, J10Hz; $-SC\underline{H}=$); m/e Found $M^+$ 367.0858, $C_{15}H_{17}N_3O_6S$ requires 367.0837; 324(8), 325(8) and 189 (4%) Found: C, 48.03; H, 4.66; N, 10.91; S, 8.61: $C_{15}H_{17}N_3O_6S$ requires C, 49.04; H, 4.66; N, 11.44; S, 8.73%.

Example 10d

p-Nitrobenzyl(5R,6R)-3-[Z-(N-2-p-nitrobenzyloxycarbonylaminoethylcarbamoyl)-ethenylthio]-6-[(S)-1-hydroxyethyl]-7-oxo-1-azabicyclo[3.2.0]hept-2-ene-2-carboxylate

a). Z-(N-2-p-nitrobenzyloxycarbonylaminoethylcarbamoyl)ethenylthioacetate (142 mg) in dioxan (7 ml) was treated with 0.1 M aqueous sodium hydroxide (7.75 ml), for 20 minutes at room temperature. The dioxan was removed to give a yellow aqueous solution.

b). p-Nitrobenzyl(5R,6R)-3-[2-acetylaminoethylsulphinyl]-6-[(S)-1-hydroxyethyl]-7-oxo-1-azabicyclo[3.2.0]hept-2-ene-2-carboxylate (90 mg) in ethyl acetate (50 ml) and cetyldimethylbenzylammonium chloride (1 mg) at 0°C was treated with the aqueous solution from a) with vigorous stirring. After 1 hour at room temperature t.l.c. analysis (ethanol:chloroform, 1:9) showed no evidence of starting material. The organic phase was separated, dried and the solvent removed. Chromatography on silica gel 60 (Merck Art. 7729; <230 ASTM) eluting with ethanol:chloroform, 1:9 gave the title compound as a colourless gum/foam, 36 mg (27%).

$\nu_{max.}$ ($CHCl_3$) 3375, 1780, 1710, 1660, 1610(w), 1525 and 1350cm$^{-1}$; $\lambda_{max.}$ (ethanol) 267 nm, 324nm, δ ($CDCl_3$) 1.43 (3H, d, J 7Hz; $-C\underline{H}_3$), 2.98 (2H, m, $-C\underline{H}_2-$), 3.18 (1H, dd, J11, 18Hz; C4-H), 3.42 (2H, m, $-C\underline{H}_2-$), 3.63 (1H, m, C6-H), 3.75 (1H, dd, J 9,18Hz;C4-H), 4.22 (1H, m, C8-H), 4.37 (1H, m, C5-H), 5.17 (2H, s, $-CO_2C\underline{H}_2Ph$), 5.36 (2H, ABq, J 14Hz; $-OCO_2C\underline{H}_2Ph$), 5.83 (1H, d, J 11Hz; $-SC\underline{H}=$), 5.98 (1H, broad s, $-N\underline{H}-$), 7.08 (1H, d, J 11Hz; $=C\underline{H}CO-$), 7.66 and 7.71 (4H, 2d, J 9Hz; arom -H), 8.18 and 8.24 (4H, 2d, J 9Hz; arom -H).

$^1$H.n.m.r. spectrum also showed presence of corresponding E-isomer of title compound in a ratio of approximately 3:2, Z:E with characteristic peaks at:- δ, 5.19 (2H, s, $-C\underline{H}_2CO_2Ph$), 5.40 (2H, ABq, J 14Hz; $-OCO_2C\underline{H}_2Ph$), 6.16 (1H, broad s, $-N\underline{H}-$), 6.23 (1H, d, J14Hz; $-SC\underline{H}=$). Other signals overlap with those of the Z-isomer.

Example 10e

E and Z isomers of (5R,6R)-3-[(N-2-aminoethylcarbamoyl)ethenylthio]-6-[(S)-1-hydroxyethyl]-7-oxo-1-azabicyclo[3.2.0]hept-2-ene-2-carboxylic acid.

10% Palladium/charcoal (15 mg) as a suspension in 50% aqueous dioxan (4 ml) was prehdrogenolysed for 20 minutes. To this was added a solution of a mixture of the E-and Z-isomers of p-nitrobenzyl (5R,6R)-3-[(N-2-p-nitrobenzyloxycarbonyl-aminoethylcarbamoyl)ethenylthio]-6-[(S)-1-hydroxyethyl]-7-oxo-1-azabicyclo[3.2.0]hept-2-ene-2-carboxylate as obtained in preparation 11, in 50% aqueous dioxan (4 ml). The total mixture was then hydrogenolysed for 2 hours. The reaction mixture was filtered through celite and the dioxan removed in vacuo. The aqueous solution was extracted with ethyl acetate (3 x 10 ml) to give a solution containing 1.33 mgs ( 5 %) of the title compound. $\lambda_{max.}$ ($H_2O$) 286 nm.

Example 11a

p-Nitrobenzyl(5R,6R)-3-[(Z)-2-isopropylaminocarbonylethenylthio]-6-[(S)-1-hydroxyethyl]-7-oxo-1-azabicyclo[3.2.0]hept-2-ene-2-carboxylate.

A solution of p-nitrobenzyl(5R,6R)-3-[(E)-2-acetamidoethenylthio]-6-[(S)-1-hydroxyethyl]-7-oxo-1-azabicyclo[3.2.0]hept-2-ene-2-carboxylate (300 mg) in 15% aqueous dioxan (25 ml) was stirred for 5 min with N-bromoacetamide (93mg). Chloroform (35 ml) was added and the solution was washed with water containing a little dilute brine. The organic solution was dried ($MgSO_4$) and evaporated *in vacuo* to afford a yellow foam.

The foam was dissolved in DMF (5 ml) and to the solution were added anhydrous potassium carbonate (93 mg) and N-isopropylpropiolamide (100 mg). The mixture was stirred at ambient temperature for 30 min before adding ethyl acetate (35 ml). The solution was washed with water (3 x 25 ml) and brine (25 ml) and then dried ($MgSO_4$). Evaporation of the solvent gave a crude product which was chromatographed on silica gel using ethyl acetate to elute. Fractions containing the product were combined and evaporated to afford a white solid which consisted of the title amide; $\lambda_{max.}$ (EtOH) 332 and 263 nm; $\nu_{max.}$ ($CH_2Cl_2$) 3590, 3430, 1780, 1710 and 1660$cm^{-1}$; δ (acetone-$d_6$) 1.16 (6H, d, J 7Hz, $Me_2CH$), 1.35 (3H, d, J 6.5Hz, MeCH), ca. 2.85 (1H, br, OH), 3.43 (1H, dd, J 10 and 18.5Hz, 4-$CH_a$), 3.63 (1H, dd, J 6 and 9Hz, 6-CH), 3.66 (1H, dd, J 10 and 18.5Hz, 4-$CH_b$), 4.04 (1H, m, $CHMe_2$), 4.17 (1H, m, CHMe), 4.37 (1H, m, 5-CH), 5.35 and 5.54 (each 1H, d, J 14Hz, $CH_2C_6H_4$-$NO_2$), 6.12 (1H, d, J 10Hz, =CHCON), 7.30 (1H, d, J 10Hz, = CH.S), 7.89 and 8.28 (each 2H, d, J 9, $CH_2C_6H_4$-$NO_2$). [$M^+$, 475].

Example 11b

Sodium (5R,6R)-3-[(Z)-2-isopropylaminocarbonylethenylthio]-6-[(S)-1-hydroxyethyl]-7-oxo-1-azabicyclo[3.2.0]hept-2-ene-2-carboxylate.

p-Nitrobenzyl(5R-6R)-3-[(Z)-2-isopropylaminocarbonylethenylthio]-6-[(S)-1-hydroxyethyl]-7-oxo-1-azabicyclo[3.2.0]hept-2-ene-2-carboxylate (10 mg) was shaken with hydrogen and 5% Pd-C catalyst (15 mg) in a mixture of dioxan (7 ml) and 0.05 M pH 7 phosphate buffer solution (3 ml). After 3 h sodium bicarbonate (3 mg) was added and the mixture was filtered over Celite and the filtrate partitioned between ethyl acetate and water. The aqueous phase was washed with two further portions of ethyl acetate, and then concentrated *in vacuo* to a volume of 25 ml. The final aqueous solution contained the title sodium salt (3 mg); $\lambda_{max.}$ ($H_2O$) 317 nm.

Example 12a

N-t-butoxycarbonyl-p-nitrobenzyl-prolinate

N-t-butoxycarbonyl-proline (0.40 g) in solution in dry dimethylformamide containing 3Å molecular sieves was treated with triethylamine (0.28 ml) and p-nitrobenzyl-bromide (0.44 g). The mixture was stirred for 60 hours. After filtration and removal of solvent under vacuum, the residue was partitioned between ethylacetate and brine. The aqueous phase was washed with ethylacetate and the combined phases dried and solvent removed to afford a yellow gum. Purification by column chromatography on silica gel 60 (Merck Art. 7729; <230 ASTM) eluting with 30% followed by 70% ethyl acetate:hexane, yielded the title compound 0.59 g (90%) as a gum.

$\nu_{max.}$ ($CHCl_3$) 1750, 1690, 1610, 1525, 1405 and 1350cm$^{-1}$; $\delta$ ($CDCl_3$) 1.31 (9H, s, t-Bu-O), 1.99 (4H, m), 3.46 (2H, m), 4.35 (1H, m), 5.29 (2H, s, $CO_2CH_2Ph$), 7.56 (2H, d, arom-H), 8.18 (2H, d, arom -H) ppm.

Example 12b

p-nitrobenzyl-prolinate hydrochloride

N-t-butoxycarbonyl-p-nitrobenzyl-prolinate (200 mg) was dissolved in acetic acid (1.5 ml) which was 1 M with respect to hydrochloric acid. After stirring for 1 hour the solution was treated with hexane (3 mls). The white solid product was collected by filtration and washed with hexane, 141 mg (86%).

$\nu_{max.}$ (Nujol mull) 1740, 1610, and 1520cm$^{-1}$; δ ($D_2O$) 2.19 (4H, m), 3.45 (2H, m), 5.43 (2H, s, $CO_2CH_2Ph$), 7.61 (2H, d, arom -H), 8.27 (2H, d, arom -H).

Example 12c

Z-2'-carboxy-pyrrolidin-1'-yl-carbonylethenyl-2-thioacetate

3-Acetylthioprop-2-enoic acid (200 mg) in dry benzene (10 ml) was treated with oxalyl chloride (240 µl) at room temperature for 60 hours. Solvent was removed and replaced with dry dichloromethane (10 ml) and solution cooled to $0^{o}C$. To this was added p-nitrobenzyl-prolinate hydrochloride (180 mg) and triethylamine (174 µl). After 30 minutes, the solution was washed with brine, dried and the solvent removed. Chromatography on silica gel 60 (Merck Art. 7729; <230 ASTM) eluting with 50% ethylacetate:hexane yielded the title compound, 166 mg (72%). This material was further purified by recrystallisation from ethylacetate:hexane.

$\nu_{max.}$ ($CHCl_3$) 1740, 1710, 1635, 1605, 1595, 1525, 1425, and 1350cm$^{-1}$; δ ($CDCl_3$) 2.09 (4H,m), 2.44 (3H, s, $\underline{CH_3}COS$), 3.69 (2H, m), 4.65 (1H, m), 5.29 (2H, s, $CO_2\underline{CH_2}Ph$), 6.35 (1H, d, J=10Hz; CO$\underline{CH}$=), 7.54 (2H, d, arom -H), 7.74 (1H, d, J=10Hz, SC$\underline{H}$=), 8.21 (2H, d, arom -H).

Example 12d

5RS,6SR-2-carboxy-3-[Z-2'-carboxy-pyrrolidin-1'-yl-carbonylethenyl-2-thio]-6-[(SR)-1-p-nitrobenzyloxycarbonyl-oxyethyl]-7-oxo-1-azabicyclo[3.2.0]hept-2-ene bis-p-nitrobenzyl ester

a) Z-2'-carboxy-pyrrolidin-1'-yl-carbonylethenyl-2-thioacetate (60 mg) in solution in dioxan (3.5 ml) was treated with 0.1 N aqueous sodium hydroxide (3.6 ml). After 15 minutes the dioxan was removed *in vacuo* and replaced with water (3.5 ml) to afford a yellow aqueous solution of the sodium thiolate.

b) p-Nitrobenzyl-3-[pyrimidin-2-ylsulphinyl]-6-[1-p-nitrobenzyloxycarbonyl-oxyethyl]-7-oxo-1-azabicyclo[3.2.0]hept-2-ene-2-carboxylate (95 mg) in dichloromethane (6 ml) was cooled to 0°C. Cetyldimethylbenzylammonium chloride (1 mg) was added, followed by the previously prepared aqueous solution from (a). The mixture was stirred vigorously at room temperature for 1.5 hours. The phases were separated and the aqueous phase washed with dichloromethane. Combined organic phases were dried and the solvent removed to afford a yellow gum. This was subjected to column chromatography on silica gel 60 (Merck Art. 7729; <230 ASTM) eluting with ethylacetate. The title compound was obtained as a cream-coloured solid after trituration with hexane. Yield 20 mg (16%).

$\nu_{max.}$ ($CHCl_3$) 1785, 1745, 1620, 1605, 1525 and 1345$cm^{-1}$; $\lambda_{max.}$ (EtOH) 266 nm, 334.5 nm.

Example 12e

5RS,6SR-2-carboxy-3-[2'-carboxy-pyrrolidin-1'-yl-carbonylethenyl-2-thio]-6-[(SR)-1-hydroxyethyl]-7-oxo-1-azabicyclo[3.2.0]hept-2-ene disodium salt

10% Palladium/charcoal catalyst (20 mg) as a suspension in 50% aqueous dioxan (10 ml) was hydrogenated for 20 minutes. To this was added 5RS,6SR-2-carboxy-3-[Z-2'-carboxy-pyrrolidin-1'-yl-carbonylethenyl-2-thio]-6-[(SR)-1-p-nitrobenzyloxycarbonyl-oxyethyl]-7-oxo-1-azabicyclo[3.2.0]hept-2-ene bis-p-nitrobenzyl ester (20 mg) in solution in dioxan (10 ml) and phosphate buffer (1 ml). The total mixture was hydrogenolysed at room temperature for 2 hours. The solution was treated with aqueous sodium hydrogen carbonate solution (0.47 ml of a 0.1 M solution) then filtered through celite. The dioxan was evaporated under vacuum and the aqueous solution extracted with ethyl acetate to give an aqueous solution containing 2 mg (20%) of the title compound. $\lambda_{max.}$ ($H_2O$) 324 nm.

DEMONSTRATION OF EFFECTIVENESS

The concentrations of compounds of the Examples required to inhibit the growth of the following bacteria are given below: MIC (μg/ml):

| ORGANISM | COMPOUND OF EXAMPLE 1 | 2 | 3 | 4 | 5 | 6 (impure) |
|---|---|---|---|---|---|---|
| Citrobacter freundii Ei | 12.5 | ≤ 0.1 | 3.1 | 0.8 | 1.6 | 50 |
| Enterobacter cloacae N1 | 50 | 0.2 | 3.1 | 1.6 | 1.6 | 25 |
| Escherichia coli 0111 | 25 | 0.2 | 0.8 | 0.2 | 0.4 | 6.2 |
| Escherichia coli JT39 | 50 | 0.2 | 0.4 | ≤ 0.1 | 0.8 | 12.5 |
| Klebsiella aerogenes A | 25 | 0.2 | 0.4 | ≤ 0.1 | 1.6 | 6.2 |
| Proteus mirabilis C977 | 50 | ≤ 0.1 | 1.6 | 0.8 | 3.1 | 50 |
| Proteus morganii 1580 | 25 | 0.8 | 6.2 | 1.6 | 6.2 | 50 |
| Proteus rettgeri WM16 | 50 | 0.4 | 3.1 | 3.1 | 6.2 | 50 |
| Proteus vulgaris W091 | 25 | 0.2 | 0.4 | 0.4 | 6.2 | 25 |
| Pseudomonas aeruginosa A | >100 | 25 | 50 | 50 | >100 | >100 |
| Salmonella typhirmurium CT10 | 25 | ≤ 0.1 | 0.4 | 0.2 | 1.6 | 6.2 |
| Serratia marcescens US20 | 25 | 0.4 | 3.1 | 0.8 | 3.1 | 12.5 |
| Shigella sonnei MB 11967 | 25 | ≤ 0.1 | 1.6 | 0.4 | 1.6 | 12.5 |
| Bacillus Subtilis A | ≤ 0.1 | ≤ 0.1 | ≤ 0.1 | ≤ 0.1 | 0.2 | 1.6 |
| Staphylococcus aureux Oxford | 0.4 | ≤ 0.1 | ≤ 0.1 | ≤ 0.1 | 0.8 | 1.6 |
| Staphylococcus aureus Russell | 0.8 | ≤ 0.1 | - | ≤ 0.1 | 1.6 | 3.1 |
| Staphylococcus aureus 1517 | 6.2 | 1.6 | 3.1 | 6.2 | 50 | 100 |
| Streptococcus faecalis I | 0.8 | 0.2 | 0.8 | 0.4 | 6.2 | 50 |
| Streptococcus pneumoniae CN33 | - | - | 0.8 | ≤ 0.1 | ≤ 0.1 | - |
| Streptococcus pyogenes CN10 | - | ≤ 0.1 | 0.8 | ≤ 0.1 | 0.2 | 3.1 |
| Escherichia coli ESS | 1.6 | ≤ 0.1 | 0.4 | ≤ 0.1 | 0.4 | 3.1 |

CLAIMS

1. A compound of formula (II):

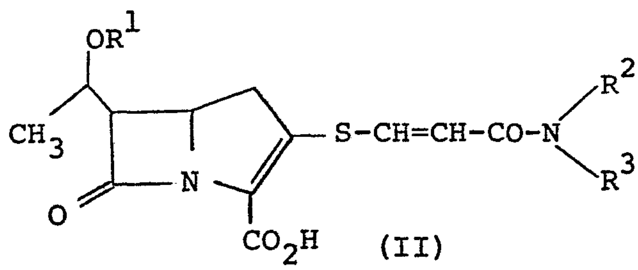

(II)

or a pharmaceutically acceptable salt or ester thereof wherein:

$R^1$ is hydrogen, -CHO, or $-SO_3H$ or a pharmaceutically acceptable salt thereof;

$R^2$ and $R^3$ are independently hydrogen, hydrocarbon, hydrocarbylcarbonyl, hydrocarbylcarbonylamino;

or $R^2$ and $R^3$ together with the nitrogen atom to which they are attached form a heterocyclic ring;

$R^2$ and $R^3$ being optionally substituted.

2. A compound as claimed in claim 1 wherein $R^2$ and $R^3$ represent hydrogen, $C_{1-6}$ alkyl, $C_{3-10}$ cycloalkyl, aryl$(C_{1-6})$alkyl, $C_{1-6}$ alkylaminocarbonyl, $C_{3-10}$ cycloalkyl$(C_{1-6})$alkyl, $C_{3-10}$ cycloalkylaminocarbonyl, halo$(C_{1-6})$alkyl, amino$(C_{1-6})$alkyl, or $C_{1-6}$ alkylcarbonyl; or $R^2$ and $R^3$ together represent $C_4$ or $C_5$ alkylene, any such groups being optionally substituted.

3. A compound as claimed in either claim 1 or 2 wherein $R^1$ represents hydrogen.

4. A compound as claimed in any one of claims 1 to 3 wherein $R^2$ is hydrogen.

5. A process for the preparation of a compound as claimed in claim 1 which process comprises:

(a) the reaction of a compound of formula (IV):

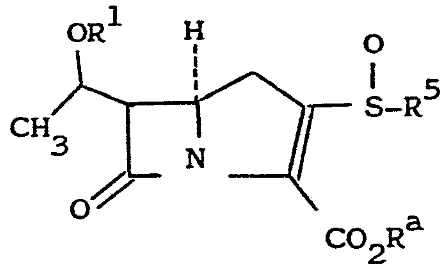

(IV)

wherein $R^1$ is as hereinbefore defined, or protected derivative thereof, $R^a$ is a carboxy-blocking group and $R^5$ is a $C_{1-6}$ alkyl, $C_{3-7}$ cycloalkyl, $C_{3-7}$ cycloalkyl $(C_{1-6})$ alkyl, aryl $(C_{1-6})$ alkyl, heterocyclyl $(C_{1-6})$ alkyl, heteroaryl $(C_{1-6})$ alkyl, heterocyclyl, heteroaryl, aryl, $C_{2-6}$ alkenyl or $C_{2-6}$ alkynyl group, any of such groups being optionally substituted; with a compound of the formula (V):

$$H-S-CH=CH-CO-NR^2R^3 \qquad (V)$$

or reactive derivative thereof wherein $R^2$ and $R^3$ are as defined in relation to formula (II); or

(b) the elimination of the elements of $R^6R^7R^8P=O$ from an ester of a compound of the formula (VI):

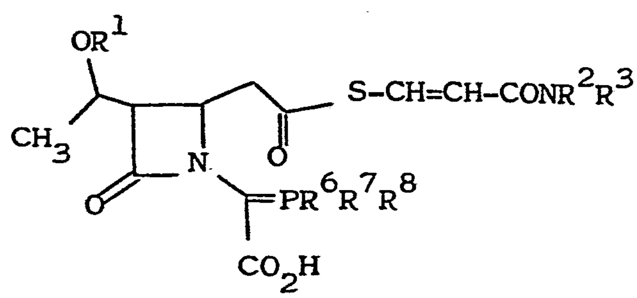

(VI)

wherein $R^1$ is as hereinbefore defined or protected derivative thereof, and $R^2$ and $R^3$ are as hereinbefore defined, and $R^6$, $R^7$ and $R^8$ are independently phenyl or methoxyphenyl; or

(c) the reaction of a compound of the formula (IX):

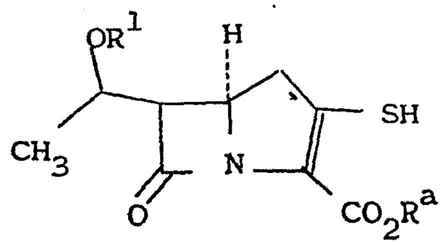

(IX)

or reactive derivative thereof, wherein $R^1$ is as hereinbefore defined or a protected derivative thereof, and $R^a$ is as hereinbefore defined; with a compound of the formula (X):

$$H\text{-}C{\equiv}C\text{-}CO\text{-}NR^2R^3 \qquad (X)$$

wherein $R^2$ and $R^3$ are as hereinbefore defined; in the presence of an acid acceptor, and after step (a), (b), or (c), optionally

i) converting a compound of the formula (II) wherein $R^1$ is -CHO to a compound of the formula (II) wherein $R^1$ is hydrogen,

ii) removing any carboxy-blocking group, or removing any protecting group,

iii) converting the product into a pharmaceutically acceptable salt or pharmaceutically acceptable ester.

6. A pharmaceutical composition which comprises a pharmaceutically acceptable carrier and a compound as claimed in any one of claims 1 to 4.

7. A composition as claimed in claim 6 which further comprises a penicillin or cephalosporin.

8. A compound as claimed in claim 1 for use in the treatment of bacterial infections.